# EUROPEAN PATENT APPLICATION

(11) **EP 4 534 145 A2**
(43) Date of publication of application: **09.04.2025**
(21) Application number: 25158891.9
(22) Date of filing: 25.04.2019
(51) Int. Cl.: A61P 15/08

(54) **COMPOSITION FOR CONTROLLED OVARIAN STIMULATION**

(30) Priority: 30.04.2018 EP 18170138; 16.05.2018 EP 18172725; 18.01.2019 EP 19152465
(62) Divisional of application: 19722535.2
(71) Applicant: Ferring B.V., 2132 JX Hoofddorp (NL)
(72) Inventor: COTTINGHAM, Ian, 1162 Saint-Prex (CH)
(74) Representative: Reddie & Grose LLP

(57) **Abstract**

Compositions including FSH and hCG for use in the treatment of infertility.

## Description

The present invention relates to compositions and pharmaceutical products for the treatment of infertility.

### Background

Assisted reproductive technologies (ART) such as in vitro fertilisation (IVF) are well known. ART generally requires a step of controlled ovarian stimulation (COS), in which a cohort of follicles is stimulated to full maturity. Standard COS regimens include administration of gonadotrophins, such as follicle stimulating hormone (FSH), alone or in combination with luteinising hormone (LH) activity to stimulate multiple follicular development. Usually COS requires administration of a GnRH analogue, or GnRH agonist, prior to and/or during stimulation to prevent a premature LH surge which may induce ovulation before planned oocyte retrieval. The pharmaceutical compositions generally used for COS include recombinant follicle stimulating hormone (rFSH) including REKOVELLE^{®} and GONAL-F^{®}, urinary derived FSH, recombinant FSH + LH preparations, urinary derived menotrophin [human menopausal gonadotrophin (hMG)] and highly purified human menopausal gonadotrophin (HP-hMG).

In case of a too high ovarian response, COS can be associated with a risk of ovarian hyperstimulation syndrome (OHSS), which can become life threatening in severe cases. The ability to predict the ovarian response potential of women to COS may allow the development of personalised or individualised COS protocols. Such individualised protocols could, for example, reduce the risk of OHSS in women predicted to have an excessive ovarian response to COS, and/or improve the chance of pregnancy in women classed as poor responders. The serum concentration of anti-Müllerian hormone (AMH) is now established as a reliable biomarker of ovarian reserve (Dewailly et al, 2014). Levels of AMH are directly correlated with the ovarian response to gonadotrophins during COS. Thus, high levels of AMH are a good predictor of excessive ovarian response, and an indicator of risk of OHSS, whereas low levels of AMH predict a poor ovarian response to COS.

Clinical research has focused the last years on the development of individualised dosing regimens for COS, initially without using AMH but based on other predictors of ovarian response. These predictors include age, body mass index (BMI), FSH and antral follicle count (AFC). As a typical example the CONSORT study applied a dosing algorithm incorporating basal FSH, BMI, age and AFC to predict the optimal rFSH starting dose for COS (Olivennes et. al., 2009). The CONSORT dosing algorithm individualises rFSH (GONAL-F^{®}) doses for ART, assigning 37.5 IU increments according to patient characteristics of: basal FSH, BMI and AFC. CONSORT was a prospective, uncontrolled, international, 18-centre, pilot study of normo-ovulatory women aged 18-34 years inclusive undergoing stimulation under a long agonist treatment protocol.. Recombinant FSH dose was assigned by the algorithm and was intended to be altered only for risk of OHSS. Primary end-point was number of oocytes retrieved. Five dose groups comprising a total of 161 patients were analysed: 75 IU (n = 48), 112.5 IU (n = 45), 150 IU (n = 34), 187.5 IU (n = 24), 225 IU (n = 10). Cancellations due to inadequate response were higher than expected in the 75 IU group (12/48). Overall, a median of 9.0 oocytes were retrieved (8.5, 8.0, 10.0, 12.0 and 8.0 in the 75, 112.5, 150, 187.5 and 225 IU groups respectively). Clinical pregnancy rates/cycle started were 31.3, 31.1, 35.3, 50.0 and 20.0%, respectively (overall, 34.2%). Two patients had severe OHSS. It was concluded that the CONSORT algorithm achieved an adequate oocyte yield and good pregnancy rates in this preliminary study. However, adjustment of the algorithm would be needed to reduce cancellation rates. Thus there is a need for more reliable individualised COS protocols which provide adequate response to stimulation, and/or decreased risk of OHSS.

As indicated above, standard COS protocols require daily FSH administration to induce multiple follicular growth to obtain sufficient oocytes for IVF. FSH is a natural hormone that is secreted by the anterior pituitary gland. In women FSH induces monthly the growth of a single dominant follicle that ovulates during each natural cycle. FSH comprises a 92 amino acid alpha sub-unit, also common to the other glycoprotein hormones LH and CG, and a 111 amino acid beta sub-unit unique to FSH that confers the biological specificity of the hormone (Pierce and Parsons, 1981). Each sub-unit is post translationally modified by the addition of complex carbohydrate residues. Both subunits carry 2 sites for N-linked glycan attachment, the alpha sub-unit at amino acids 52 and 78 and the beta sub-unit at amino acid residues 7 and 24 (Rathnam and Saxena, 1975, Saxena and Rathnam, 1976). FSH is thus glycosylated to about 30% by mass (Dias and Van Roey. 2001. Fox *et al.* 2001)*.*

FSH purified from the urine of post-menopausal women has been used for many years in infertility treatment; both to promote ovulation in natural reproduction and to induces multiple follicular growth to obtain sufficient oocytes for ART. Until recently, the only approved rFSH products for ovarian stimulation, such as follitropin alfa (GONAL-F^{®}, Merck Serono / EMD Serono) and follitropin beta (PUREGON^{®} / FOLLISTIM^{®}, MSD / Schering-Plough), were derived from a Chinese Hamster Ovary (CHO) cell line.

There is considerable heterogeneity associated with FSH preparations which relates to differences in the amounts of various isoforms present. Individual FSH isoforms exhibit identical amino acid sequences but differ in the extent of their glycosylation. These particular isoforms may be characterised by their heterogeneity of the carbohydrate branch structures and differing amounts of sialic acid (a terminal sugar) incorporation, both of which appear to influence the specific isoform bioactivity.

Glycosylation of natural FSH is highly complex. The glycans in naturally derived pituitary FSH can contain a wide range of structures that can include combinations of mono-, bi-, tri- and tetra-antennary glycans (Pierce and Parsons, 1981. Ryan *et al.,* 1987. Baenziger and Green, 1988). The glycans can carry further modifications: core fucosylation, bisecting glucosamine, chains extended with acetyl lactosamine, partial or complete sialylation, sialylation with α2,3 and α2,6 linkages, and sulphated galactosamine substituted for galactose (Dalpathado *et al.,* 2006). Furthermore, there are differences between the distributions of glycan structures at the individual glycosylation sites. A comparable level of glycan complexity has been found in FSH derived from the serum of individuals and from the urine of post-menopausal women (Wide *et* al., 2007).

The glycosylation of rFSH products reflects the range of glycosyl-transferases present in the host cell line. Commercially available rFSH products derived from engineered CHO cells have a more limited range of glycan modifications than those found on the natural products. Examples of the reduced glycan heterogeneity found in CHO cell derived rFSH include a lack of bisecting glucosamine and a reduced content of core fucosylation and acetyl lactosamine extensions (Hard *et al.,* 1990). In addition, CHO cells are only able to add sialic acid using the α2,3 linkage (Kagawa *et al,* 1988, Takeuchi *et al,* 1988, Svensson *et al.,* 1990); CHO cell derived rFSH only includes α2,3-linked sialic acid and does not include α2,6-linked sialic acid.

Thus CHO cell derived FSH is different from naturally produced FSH (e.g. human pituitary/ serum/ urinary FSH) which contains glycans with a mixture of α2,3 and α2,6-linked sialic acid, with a predominance of the former.

The present applicants have developed a human cell line derived rFSH which is the subject of International Patent Application No. PCT/GB2009/000978, published as WO2009/127826A. Recombinant FSH with a mixture of both α2,3 and α2,6-linked sialic acid was made by engineering a human cell line to express both rFSH and α2,3 sialyltransferase. The expressed product is highly acidic and carries a mix of both α2,3- and α2,6-linked sialic acids; the latter provided by the endogenous sialyl transferase activity. It was found that the type of sialic acid linkage, α2,3- or α2,6-, can have a dramatic influence on biological clearance of FSH. Recombinant FSH with a mixture of both α2,3 and α2,6-linked sialic acid has two advantages over rFSH expressed in conventional CHO cells: first the material is more highly sialylated due to the combined activities of the two sialyltransferases; and secondly the material more closely resembles the natural FSH. This is likely to be more biologically appropriate compared to CHO cell derived recombinant products that have only α2,3 linked sialic acid (Kagawa *et al,* 1988, Takeuchi *et al,* 1988, Svensson *et al.,* 1990) and have decreased sialic acid content (Ulloa-Aguirre *et al.* 1995., Andersen *et al.* 2004)*.*

The amino acid sequence of the human cell line derived recombinant FSH which is the subject of International Patent Application No. PCT/GB2009/000978, published as WO2009/127826A, is the native sequence and is identical to natural human FSH and existing CHO-derived rFSH products. However, the present applicants have found that human derived recombinant FSH products (i.e. recombinant FSH produced or expressed in a human cell line e.g. made by engineering a human cell line) which have a mixture of both α2,3 and α2,6-linked sialic acid may be particularly effective when utilised in (e.g. individualised) COS protocols.

On 13 December 2016, the European Commission (EC) granted marketing authorisation for REKOVELLE^{®} (follitropin delta, also known as FE 999049), a human cell line derived recombinant follicle stimulating hormone (human rFSH), for use in controlled ovarian stimulation for the development of multiple follicles in women undergoing assisted reproductive technologies (ART), such as an *in vitro* fertilisation (IVF) cycle. REKOVELLE^{®} is the first rFSH to be derived from a human cell line. The REKOVELLE^{®} (follitropin delta) product is produced by the methods disclosed in International Patent Application No. PCT/GB2009/000978.

Two randomised, controlled, assessor-blind, parallel groups, multi-centre phase 2 anti-Müllerian hormone (AMH)-stratified trials were conducted in IVF/ICSI patients, one in Europe and one in Japan, with the purpose of determining the dose-response relationship of FE 999049 and the number of oocytes retrieved. In both trials, randomisation was stratified according to AMH levels at screening; low AMH (5.0-14.9 pmol/L) or high AMH (15.0-44.9 pmol/L). In the European dose-response phase 2 trial, five doses of FE 999049 ranging from 5.2 µg/day to 12.1 µg/day were investigated and a reference group of an approved rFSH product (GONAL-F^{®}, 150 IU/day) was also included. In the Japanese dose-response phase 2 trial, three doses of FE 999049 (6 µg/day, 9 µg/day and 12 µg/day) were investigated and a standard therapy of the approved rFSH product (FOLLISTIM^{®}, 150 IU/day) was also included. At present, follitropin beta (FOLLISTIM^{®}) is the only medicinal product approved in Japan for controlled ovarian stimulation in IVF/ICSI cycles.

In the European and the Japanese phase 2 trials, the daily dose was fixed throughout the stimulation period. In both trials, a statistically significant dose response relationship for FE 999049 with respect to the number of oocytes retrieved was observed for the overall population and for each AMH randomisation stratum. Acceptable pregnancy rates were achieved with all FE 999049 doses. Furthermore, the observed FE 999049 dose-response profile was similar in the European trial and in the Japanese trial.

This work enabled the development of individualised COS protocols for dosing the REKOVELLE^{®} (follitropin delta, FE 999049) product.

The applicants have previously found that it is generally necessary to retrieve in the region of nine oocytes in order to enable selection of two high quality embryos for transfer.

The applicants have found that for subjects having low AMH (AMH < 15 pmol/L per litre) a reasonably high dose of follitropin delta is required (for example 12 µg) to achieve this. At this dose, 8 to 14 oocytes will be retrieved from 60% of subjects with low AMH. This is an unexpected and significant improvement over treatment of subjects with low AMH treated with 150 IU GONAL-F^{®}, where 8 to 14 oocytes are retrieved from only 33% of subjects. The applicants have found that there is no need to adjust this dose according to the bodyweight of the patient.

However, 60 % of the population (and 80% of women under 30 treated for infertility) have high AMH (that is, AMH of ≥15 pmol/L). For these subjects it is generally fairly straightforward to retrieve a mean of 9 to 11 oocytes; the problem with stimulation protocols is the risk of OHSS. The applicants have found that in patients dosed at low doses of follitropin delta there is a relationship between oocytes retrieved and body weight of the subject. This means that there may be a risk associated with treatment with a fixed dose of FSH (which is usual in the art). The present applicants have established a relationship between dose of FSH and AMH level and weight of the subject which provides an improved safety profile (reduced risk of OHSS) with acceptable or improved number of oocytes retrieved compared conventional treatment protocols.

The posology of REKOVELLE^{®} is individualised for each patient and aims to obtain an ovarian response which is associated with a favourable safety/efficacy profile, i.e. aims to achieve an adequate number of oocytes retrieved and reduce the interventions to prevent OHSS. REKOVELLE^{®} is dosed in micrograms.

For the first treatment cycle, the individual daily dose will be determined on the basis of the woman's serum AMH concentration and her body weight. The dose should be based on a recent determination of AMH (i.e. within the last 12 months) measured by the following diagnostic test from Roche: ELECSYS^{®} AMH Plus immunoassay. The individual daily dose is to be maintained throughout the stimulation period.

For women with AMH <15 pmol/L the daily dose of REKOVELLE^{®} is 12 micrograms, irrespective of body weight.

For women with AMH ≥15 pmol/L the daily dose of REKOVELLE^{®} decreases from 0.19 to 0.10 micrograms/kg by increasing AMH concentration (Table A, below).

The dose is to be rounded off to the nearest 0.33 micrograms to match the dosing scale on the injection pen. The maximum daily dose for the first treatment cycle is 12 micrograms. For calculation of the REKOVELLE^{®} dose, the body weight is to be measured without shoes and overcoat just prior to start of stimulation.

**Table A Dosing regimen**

| | | | | | | | | | | | |
|---|---|---|---|---|---|---|---|---|---|---|---|
| AMH (pmol/L) | <15 | 15-16 | 17 | 18 | 19-20 | 21-22 | 23-24 | 25-27 | 28-32 | 33-39 | ≥40 |
| Fixed daily dose of REKOVELLE^{®} | 12.0 mcg | 0.19 mcg/kg | 0.18 mcg/kg | 0.17 mcg/kg | 0.16 mcg/kg | 0.15 mcg/kg | 0.14 mcg/kg | 0.13 mcg/kg | 0.12 mcg/kg | 0.11 mcg/kg | 0.10 mcg/kg |
| mcg: micrograms | | | | | | | | | | | |

The AMH concentration is to be expressed in pmol/L and is to be rounded off to the nearest integer. If the AMH concentration is in ng/mL, the concentration should be converted to pmol/L by multiplying with 7.14 (ng/mL x 7.14 = pmol/L) before use.

Treatment with REKOVELLE^{®} should be initiated day 2 or 3 after start of menstrual bleeding. Treatment will continue until adequate follicular development (≥3 follicles ≥17 mm) has been achieved, which on average is by the ninth day of treatment (range 5 to 20 days). A single injection of 250 micrograms recombinant human chorionic gonadotropin (hCG) or 5,000 IU hCG is administered to induce final follicular maturation. In patients with excessive follicular development (of ≥25 follicles ≥12 mm), treatment with REKOVELLE^{®} should be stopped and triggering of final follicular maturation with hCG should not be performed.

For subsequent treatment cycles, the daily dose of REKOVELLE^{®} should be maintained or modified according to the patient's ovarian response in the previous cycle. If the patient had adequate ovarian response in the previous cycle without developing OHSS, the same daily dose should be used. In case of ovarian hypo-response in the previous cycle, the daily dose in the subsequent cycle should be increased by 25% or 50%, according to the extent of response observed. In case of ovarian hyper-response in the previous cycle, the daily dose in the subsequent cycle should be decreased by 20% or 33%, according to the extent of response observed. In patients who developed OHSS or were at risk of OHSS in a previous cycle, the daily dose for the subsequent cycle is 33% lower than the dose the cycle where OHSS or risk of OHSS occurred. The maximum daily dose is 24 micrograms.

The efficacy and safety of the REKOVELLE^{®} individualised dosing regimen based on the woman's serum AMH and body weight has been confirmed in a large phase 3 trial, ESTHER-1 (Evidence based Stimulation Trial with Human rFSH in Europe and Rest of World), conducted in 11 countries including Europe, North America and Latin America. The ESTHER-1 trial was conducted in 1,326 IVF/ICSI patients who were randomised 1:1 to controlled ovarian stimulation with one of the following treatments: 1) FE 999049 in its individualised dosing regimen with the daily dose fixed throughout simulation, or 2) an approved CHO-derived rFSH product (follitropin alfa, GONAL-F^{®}) at a standard starting dose of 150 IU/day followed by dose adjustments based on the subject's follicular response during stimulation. FE 999049 in its individualised dosing regimen was demonstrated to be non-inferior to follitropin alfa with respect to ongoing pregnancy rate (30.7% versus 31.6%) and ongoing implantation rate (35.2% versus 35.8%). For the overall population, there was no statistically significant difference between treatment groups in terms of number of oocytes retrieved, with an average of 10.0 for FE 999049 and 10.4 for follitropin alfa. Nevertheless, the individualised FE 999049 dosing regimen in comparison to follitropin alfa led to statistically significantly more oocytes retrieved among patients with AMH <15 pmol/L (population at risk of hyporesponse) with an average of 8.0 versus 7.0 and statistically significantly fewer oocytes among patients with AMH ≥15 pmol/L (population at risk of hyperresponse) with an average of 11.6 versus 13.3. The immediate clinical relevance of this shift in ovarian response with FE 999049 therapy was realised as statistically significantly fewer patients with extreme ovarian response compared to follitropin alfa, i.e. <4 oocytes among patients with AMH <15 pmol/L (12% versus 18%) and ≥15 or ≥20 oocytes among patients with AMH ≥15 pmol/L (28% versus 35%, and 10% versus 16%). The percentage of patients with an appropriate ovarian response, defined for FE 999049 as 8-14 oocytes, was reached by more patients treated with FE 999049 compared to follitropin alfa, i.e. 43% versus 38%, despite implementation of dose adjustments during stimulation for 37% of the patients in the follitropin alfa group in contrast to the fixed-dose individualised dosing regimen for FE 999049. A statistically significantly lower total gonadotropin dose in the FE 999049 group compared to the CHO-derived rFSH product group was observed with an average of 90 µg and 104 µg, respectively.

The most serious risk associated with gonadotropin treatment is ovarian hyperstimulation syndrome (OHSS). Overall, in the ESTHER-1 phase 3 trials, OHSS and/or preventive interventions of early OHSS occurred in 4.4% of the FE 999049 cycles and 6.5% of the follitropin alfa cycles. Moderate/severe OHSS and/or preventive interventions for early OHSS were observed at an incidence of 3.3% and 5.6% of the treatment cycles with FE 999049 and follitropin alfa, respectively.

As explained above, COS protocols may also use FSH and LH activity, and the latter may be in the form of human chorionic gonadotropin (hCG). Human chorionic gonadotropin (hCG) is a natural glycoprotein produced by the human placenta. During a normal pregnancy; hCG secreted by the placenta maintains corpus luteum after LH secretion decreases, supporting continued secretion of oestrogen and progesterone to ensure embryo implantation and development of the early pregnancy.. During the normal menstrual cycle, LH supports FSH in the development and maturation of the dominant follicle, and since hCG is also an agonist of the LH receptor, it can substitute for LH in this role.

HCG comprises a 92 amino acid alpha sub-unit, also common to the other glycoprotein hormones LH and FSH, and a 145 amino acid beta sub-unit unique to hCG, which dictates the hormone specificity. Each sub-unit is post translationally modified by the addition of complex carbohydrate residues. The alpha sub-unit contains 2-N-linked glycosylation sites at amino acids 52 and 78 and the beta sub-unit contains 2-N-linked glycosylation sites at amino acids 13 and 30 and four O-linked glycosylation sites at amino acids 121, 127, 132 and 138.

HCG extracted from the urine of pregnant women [CHORAGON^{®} (Ferring)] has been used for many years in infertility treatment. The production of hCG extracted from urine involves the collection and processing of large amounts of urine. A recombinant version of hCG, OVITRELLE^{®} (Merck Serono), is also available and expressed in CHO cells.

The present applicants have developed a human cell line derived recombinant hCG which is the subject of International Patent Application No. PCT/GB2010/001854, published as WO2011/042688A. Recombinant hCG with a mixture of both α2,3 and α2,6-linked sialic acid was made by engineering a human cell line to express both rhCG and α2,3 sialyltransferase. The expressed product is highly acidic and carries a mix of both α2,3- and α2,6-linked sialic acids; the latter provided by the endogenous sialyl transferase activity. Recombinant hCG with a mixture of both α2,3 and α2,6-linked sialic acid has two advantages over rhCG expressed in conventional CHO cells: first the material is more highly sialylated due to the combined activities of the two sialyltransferases; and secondly the material more closely resembles the natural hCG.

FE 999302 is a human cell line derived recombinant hCG produced by the method described in International Patent Application No. PCT/GB2010/001854. FE 999302 is being developed by Ferring Pharmaceuticals and intended for treatment of infertility given as daily administrations in e.g. combination with rFSH. FE 999302 is produced in the same human cell line as FE999049 (follitropin delta, REKOVELLE^{®}). This is a human derived cell line, PER.C6^{®}, differentiating it from OVITRELLE^{®}, a recombinant hCG produced from CHO cells. The PER.C6^{®} cell line has been generally commercially available for many years. Further, the Per.C6 cell was deposited under the accession number 96022940 with the European Collection of Cell Cultures ECACC, UK. The European patent application which refers to the deposited biological material is EP 1 445 3322 A1 filed by Crucell Holland B.V. on June 14, 1996. The cell line was available by request under Rule 33 EPC at the present filing date, as well as commercially available.

The doses of FE 999302 administered are specified by mass since that is intended for the final product. A phase I trial showed that single subcutaneous administration of 4-256 µg FE 999302, and repeated administration of 8 µg/day and 16 µg/day FE 999302, were safe and well tolerated and did not cause any safety concerns. FE 999302 demonstrated improved pharmacokinetics (AUC) over a CHO cell derived hCG product.

Following successful development of the individualised follitropin delta treatment (REKOVELLE^{®}) described above, the applicants developed a combined formulation including both rFSH (REKOVELLE^{®}, FE 999049, follitropin delta) and rhCG (FE 999302) in an optimal treatment ratio. In protocols where FSH and hCG are administered together it is advantageous to have the two active ingredients in the same preparation, to minimise the number of injections for the patient. However, where the dosing is individualised (i.e. the dose of a drug is specifically tailored to the individual patient, so different patients may receive markedly different doses) it can be difficult to provide a single formulation including both FSH and hCG which is suitable for each and every patient. For example, the amount of FSH may be individualised according to the protocol above, but inclusion of hCG in the same preparation with a fixed ratio of FSH:hCG may mean, depending on the amount of FSH administered, that the amount of hCG is too low (ineffective) or too high (ineffective and/or liable to lead to potentially serious side effects).

The present applicants have developed single compositions including both FSH and hCG with a ratio of FSH and hCG which provides effective and safe dosing of FSH and hCG whatever the AMH level and bodyweight of the patient. The claimed compositions allow, for example, individualised dosing of FSH based on AMH and, depending on patients's AMH level, their bodyweight, together with a dose of hCG which is pharmaceutically effective. It is believed that the phase II clinical trial proposed in Example 1 will demonstrate that administration of FSH and hCG in the specified doses and ratios is particularly effective, e.g. in terms of improved blastocyst and/or embryo quality (e.g. improved development of category 3BB or better blastocysts). More good quality blastocysts enable more frozen embryo transfers and therefore a higher cumulative (i.e. combined fresh and frozen) pregnancy rate. Blastocyst quality is regarded as the best indicator for designing a phase 3 trial where the desired outcome is an increase in pregnancy and/or cumulative pregnancy rates. It is believed that improved embryo implantation may also result from administration of FSH and hCG in the specified doses and ratios. The claimed compositions may also be effective (e.g. at higher doses) to treat patients who had insufficient ovarian response in a previous treatment (e.g. a previous COS cycle), for example with reduced risk of OHSS.

It is presently preferred that the composition will include FE999302, a human (PERC6^{®}) cell line derived recombinant hCG produced by the method described in International Patent Application No. PCT/GB2010/001854. However, it will be appreciated that other hCG products such as OVITRELLE^{®} and CHORAGON^{®} may be used in the composition.

### Description of the Invention

According to the present invention in a first aspect there is provided a composition comprising recombinant follicle stimulating hormone (FSH) and recombinant human chorionic gonadotropin (hCG) for use in the treatment of infertility in a patient having serum AMH level <15 pmol/L, wherein the recombinant FSH is to be administered at a dose of, or equivalent to, 11 to 13 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, , for example 3:1 or 1.5:1. The ratio (daily dose of rFSH in µg: daily dose of rhCG in µg) may be, for example, 12:1, 6:1, 3:1, 2:1, 1.5:1, 1:1 etc..

Preferably the composition is a single composition (e.g. a single pharmaceutical composition) comprising FSH and hCG.

The composition may be for use wherein the recombinant FSH is to be administered at a dose of 11 to 13 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example, 12:1, 6:1, 3:1, 2:1, 1.5:1, 1:1 etc..

The composition may be for use wherein the ratio by weight of the dose per day of recombinant FSH to the dose per day of recombinant hCG (daily dose of rFSH in µg: daily dose of rhCG in µg) is 3:1 or 1.5:1.

According to the present invention in a further aspect, there is provided a composition comprising recombinant follicle stimulating hormone (FSH) and recombinant human chorionic gonadotropin (hCG) for use in the treatment of infertility in a patient having serum AMH level <15 pmol/L, wherein the recombinant FSH is to be administered at a dose of 11 to 13 µg, for example 12 µg, per day, and the recombinant hCG is to be administered such that the ratio by weight of the dose per day of recombinant FSH to the dose per day of recombinant hCG (daily dose of rFSH in µg: daily dose of rhCG in µg) is 3:1 or 1.5:1 [e.g. a daily dose of 12 µg rFSH with a daily dose of rhCG of 4 µg (3:1) or a daily dose of 12 µg rFSH with a daily dose of rhCG of 8 µg (1.5:1)].

The recombinant FSH and recombinant hCG may be administered at doses per day of 12 µg recombinant FSH with 4 µg recombinant hCG; or 12 µg recombinant FSH with 8 µg recombinant hCG.

The treatment of infertility may comprise a step of determining the serum AMH level of the patient, and a step of administering the doses of recombinant FSH and recombinant hCG to a patient having serum AMH level of <15 pmol/L. The step of identifying the patient (prior to treatment) based on the serum AMH level of the patient may take place just before (e.g. 0 to 2 days before) the doses of FSH and hCG are first administered to the patient. The step of determining the serum AMH level of the patient may take place up to twelve months before the dose is first administered to the patient. Preferably the serum AMH level of the patient is determined (measured) by the ELECSYS^{®} AMH Plus immunoassay (available from Roche, of Switzerland, see www.roche.com).

According to the present invention in a further aspect there is provided a composition comprising recombinant follicle stimulating hormone (FSH) and recombinant human chorionic gonadotropin (hCG) for use in the treatment of infertility in a patient having serum AMH level of ≥15 pmol/L, wherein the composition is to be administered at a dose of, or equivalent to, 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 12:1, 6:1, 3:1 or 1.5:1. The ratio (daily dose of rFSH in µg: daily dose of rhCG in µg) may be, for example, 12:1, 6:1, 3:1, 2:1, 1.5:1, 1:1 etc..

Preferably the composition is a single composition (e.g. a single pharmaceutical composition) comprising FSH and hCG.

The composition may be for use wherein the composition is to be administered at a dose of 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example, 12:1, 6:1, 3:1, 2:1, 1.5:1, 1:1 etc..

The composition may be for use wherein the dose per day of recombinant FSH is from a minimum dose of 6 µg to a maximum dose of 12 µg.

The composition may be for use wherein the ratio by weight of the dose per day of recombinant FSH to the dose per day of recombinant hCG (daily dose of rFSH in µg: daily dose of rhCG in µg) is 3:1 or 1.5:1.

According to the present invention in an aspect there is provided a composition comprising recombinant follicle stimulating hormone (FSH) and recombinant human chorionic gonadotropin (hCG) for use in the treatment of infertility in a patient having serum AMH level of ≥15 pmol/L, wherein the composition is to be administered at a dose of 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is 3:1 or 1.5:1.

The composition may be for use in (i) the treatment of a patient having serum AMH level of 15 to 24.9 pmol/L, wherein the recombinant FSH is to be administered at a dose of 0.14 to 0.19 µg per kg bodyweight of the patient per day; or (ii) for use in the treatment of a patient having serum AMH level of 25 to 34.9 pmol/L, wherein the recombinant FSH is to be administered at a dose of 0.11 to 0.14 µg per kg bodyweight of the patient per day; or (iii) for use according in the treatment of a patient having serum AMH level of ≥ 35 pmol/L, wherein the recombinant FSH is to be administered at a dose of 0.10 to 0.11 µg recombinant FSH per kg bodyweight of the patient per day.

The treatment of infertility may include a step of identifying the patient (prior to treatment) based on the serum AMH level. The treatment of infertility may include a step of administering the defined doses of FSH and hCG to the patient identified as having the defined serum AMH level. For example, the treatment of infertility may include a step of identifying the patient (prior to treatment) based on the serum AMH level and bodyweight of the patient, and a step of administering the dose to the patient identified (prior to treatment) as having AMH ≥ 15pmol/L (for example AMH ≥ 16 pmol/L, for example AMH ≥ 19 pmol/L, for example AMH ≥ 26 pmol/L, for example AMH ≥ 28 pmol/L, for example AMH ≥ 40 pmol/L).

The step of identifying the patient (prior to treatment) based on the serum AMH level and bodyweight of the patient may take place just before (e.g. 0 to 2 days before) the dose is first administered to the patient. The step of identifying the patient may be based on a serum AMH level determined previously (e.g. a serum AMH level determined up to twelve months before the dose is first administered to the patient). Preferably the serum AMH level of the patient is determined (measured) by the ELECSYS^{®} AMH Plus immunoassay (available from Roche, of Switzerland, see www.roche.com). The bodyweight of the patient may be determined just before (e.g. 0 to 2 days before) the dose is first administered to the patient. The step of determining the bodyweight of the patient may use weighing scales, as are well known.

Preferably the rFSH (e.g. human cell line derived recombinant FSH) includes α2,3- and α2,6- sialylation. The rFSH for use according to the invention may have 1% to 99% of the total sialylation being α2,3-sialylation. The rFSH for use according to the invention may have 1% to 99% of the total sialylation being α2,6-sialylation. The recombinant FSH may have 1 % to 50% of the total sialylation is α2, 6-sialyation, and 50% to 99% of the total sialylation is α 2,3-sialyation. Preferably, 80 to 95%, for example 80 to 90%, for example 82 to 89%, for example 85 to 89% of the total sialylation is α2,3-sialylation. Preferably 5 to 20%, for example 10 to 20 %, for example 11 to 18%, for example 11 to 15%, of the total sialylation is α2,6- sialylation.

Preferably the recombinant hCG includes α2,6-sialylation and α2,3-sialylation. The rhCG for use according to the invention may have 1% to 99% of the total sialylation being α2,3-sialylation. The rhCG for use according to the invention may have 1% to 99% of the total sialylation being α2,6-sialylation. The recombinant hCG may have 1 % to 50% of the total sialylation is α2, 6-sialyation, and 50% to 99% of the total sialylation is α 2,3-sialyation. The rhCG may preferably further include tetra (4S) sialylated structures. The rhCG may preferably further include mono (1S), di (2S), tri (3S) and tetra (4S) sialylated structures. Mono (1S), di (2S), tri (3S) and tetra (4S) sialylated structures are well known in the art, e.g. as described in WO2011/042688.

Herein, by "sialylation", it is meant the amount of sialic residues present on the FSH (or hCG) carbohydrate structures. α2,3-sialylation means sialylation at the 2,3 position (as is well known in the art) and α2,6 sialylation at the 2,6 position (also well known in the art). Thus "% of the total sialylation may be α 2,3 sialylation" refers to the % of the total number of sialic acid residues present in the FSH (or hCG) which are sialylated in the 2,3 position. The term "% of the total sialylation being α2,6-sialylation" refers to the % of the total number of sialic acid residues present in the FSH (or hCG) which are sialylated in the 2,6 position. The rFSH and/or hCG may be present as a single isoform or as a mixture of isoforms.

The patient is a female patient. The patient may be over 30 years of age, optionally over 37 years of age. The patient may have previously failed at least one cycle of infertility treatment. In a specific example, the composition is for treatment of a patient over 30 years of age, the patient having have previously failed at least one cycle of infertility treatment. The patient may have previously failed up to three cycles of infertility treatment.

The treatment of infertility may be to promote good quality blastocysts (e.g. category 3BB or higher blastocysts, e.g. treatment of infertility to increase the number of category 3BB or higher blastocysts on day 3 and/or day 5 after oocyte retrieval) and/or to improve embryo implantation. Herein a "good-quality blastocyst" is defined as category 3BB or higher (i.e. 3BB, 3BA, 3AC, 3AB, 3AA, 4CC, 4CB,4CA, 4BC, 4BB, 4BA, 4AC, 4AB, 4AA, 5CC, 5CB, 5CA, 5BC, 5BB, 5BA, 5AC, 5AB, 5AA, 6CC,6CB, 6CA, 6BC, 6BB, 6BA, 6AC, 6AB, 6AA). The treatment of infertility may be treatment of infertility to increase the number of category 3BB or higher blastocysts on 3 and/or day day 5 after oocyte retrieval (e.g. compared to treatment with Rekovelle^{®} or GONAL-F^{®}, see e.g. Example 1).). The treatment of infertility may be treatment of infertility to increase the number of fertilised (2PN) oocytes (e.g. compared to treatment with Rekovelle^{®} or GONAL-F^{®}).

According to the present invention in a further aspect there is provided a (e.g. single) pharmaceutical composition comprising recombinant follicle stimulating hormone (FSH) and recombinant human chorionic gonadotropin (hCG) wherein the ratio by weight of the recombinant FSH to the recombinant hCG ( amount of rFSH in µg: amount of rhCG in µg) is 3:1 or 1.5:1.

Herein, "day one of treatment", also referred to as "day one of stimulation", refers to the first day that the dose of (e.g. recombinant) FSH and (e.g. recombinant) hCG is administered to the patient. Day one of treatment (stimulation) may take place on day 1, 2 or 3, preferably day 2 or day 3, of the patient's menstrual cycle. In other words, day one of treatment (stimulation) may be one, two or three days, preferably two or three days, after the patient commences menstrual bleeding, as is applied in clinical practice with GnRH antagonist or GnRH agonist protocols.

The doses of FSH and hCG start on day one of treatment and may continue for two to twenty days, for example continue for 2, 3, 4, 5, 6, 7, 8, 9, 10, 11, 12, 13, 14, 15, 16, 17, 18, 19, or 20 days. The doses of FSH and hCG start on day one of treatment and may continue for seven to thirteen days, for example nine to thirteen days, for example 10 to 13 days, for example 10 to 11 days. The doses of FSH and hCG may be administered at a dose equivalent to the daily doses mentioned above. For example, the composition may be for administration at daily doses of 12 µg FSH and 8 µg hCG, or an equivalent of 36 µg FSH and 24 µg hCG every three days (e.g. for administration on days 1, 4, 7 and so on).

The composition (e.g. pharmaceutical composition) or medicament may be administered after pre-treatment of the patient with a (different) pharmaceutical composition which suppresses endogenous gonadotropin production prior to day one of the treatment with FSH and hCG (e.g. after the subject has been (pre-)treated with a steroid, a GnRH agonist, a GnRH antagonist etc.). Herein, the term "pre-treated" or "pre-treatment" refers to administration of the pharmaceutical composition which suppresses endogenous gonadotropin production prior to day one of the treatment with FSH and hCG, as is applied in clinical practice with long GnRH agonist protocols. Thus, the composition (e.g. pharmaceutical composition) or medicament may be for administration 12 to 16, e.g. 13 to 15, e.g. 14 days after administration of (e.g. after initiation of administration of, e.g. after initiation of daily administration of) a GnRH agonist (e.g. SYNAREL^{®}, LUPRON^{®}, DECAPEPTYL^{®}). The composition may be for administration with a GnRH agonist.

In other examples, the composition (e.g. pharmaceutical composition) or medicament may be for administration prior to administration of a GnRH antagonist (e.g. GANIRELlX^{®}, CETRORELIX^{®}), for example for administration five or six days prior to administration of a GnRH antagonist. The product may be for administration with a GnRH antagonist.

Preferably the composition (e.g. pharmaceutical composition) or medicament is for administration prior to administration of a high dose of hCG (for example 4,000 to 11,000 IU hCG, e.g. 5,000 IU hCG, 10,000 IU hCG etc.; or 150 to 350 microgram recombinant hCG, for example 250 microgram recombinant hCG) to induce final follicular maturation.

The doses listed above may be for treatment of infertility in the patient's (subject's) first stimulation protocol. It will be appreciated that for further stimulation cycles, the doses may be adjusted according to actual ovarian response in the first cycle.

In all aspects it is preferred that the treatment of infertility, is or includes, a step of COS. Preferably the patient is a female patient (men do not have ovaries). The cause of infertility could be the woman's partner suffering from male infertility, although it will be appreciated that it is the woman who is treated by COS.

The applicants have devised "individualised" COS protocols wherein specific doses of recombinant FSH having specific characteristics are used to treat patients based on their specific AMH levels, together with an effective amount of hCG, thereby increasing the likelihood of adequate response to stimulation (e.g. in patients having a low response potential), and/or decreased risk of OHSS (e.g. in patients classed as high or excessive responders).

The serum level of AMH may be determined (e.g. measured) by any validated AMH assay. The serum AMH level may be measured using the AMH Gen-II enzyme linked immunosorbent assay, a kit (Beckman Coulter, Inc., Webster, Texas). The serum AMH level may be measured using the automated AMH ACCESS^{®} assay (Beckman Coulter, Inc., Webster, Texas). Preferably, the serum AMH level is measured using the ELECSYS^{®} AMH assay from Roche Diagnostics. Other assays may be used.

Herein, serum AMH values are generally recited in terms of pmol/L. This may be converted to ng/mL using the conversion equation 1ng/ml AMH = 7.1 pmol/L AMH.

Herein the terms "patient" and "subject" are used interchangeably.

Herein the term "treatment of infertility" includes treatment of infertility by controlled ovarian stimulation (COS) or methods which include a step or stage of controlled ovarian stimulation (COS), for example in vitro fertilisation (IVF), or intracytoplasmic sperm injection (ICSI). The term "treatment of infertility" includes treatment of infertility in a subject having tubal or unexplained infertility, including treatment of infertility in a subject having endometriosis, for example stage I or stage II endometriosis, and/or in a subject having anovulatory infertility, for example WHO type II anovulatory infertility, and/or in a subject with a partner with male factor infertility. The product (or composition) may be for (use in) the treatment of infertility (and/or for controlled ovarian stimulation) in a subject having endometriosis, for example in a subject having stage I or stage II endometriosis, as defined by The American Society for Reproductive Medicine (ASRM) classification system for the various stages of endometriosis, (stage IV most severe; stage I least severe) [American Society for Reproductive Medicine. Revised American Society for Reproductive Medicine classification of endometriosis: 1996. Fertil Steril 1997; 67,817 821.].

The composition or medicament may be for (use in) the treatment of infertility (and/or for controlled ovarian stimulation) in a subject having normal serum FSH level of 1 to 16 IU/L, for example 1 to 15 IU/L, for example 1 to 12 IU/L in the early follicular phase.

The composition or medicament may be for (use in) the treatment of infertility (and/or for controlled ovarian stimulation) in a subject aged 18 to 42 years, for example 25 to 37 years or 30 to 42 years. The product may be for (use in) the treatment of infertility (and/or for controlled ovarian stimulation) in a subject having BMI >1 and BMI < 35 kg/m², for example a subject having BMI >18 and BMI < 32 kg/m², for example a subject having BMI >18 and BMI < 25 kg/m², for example a subject having BMI >20 and BMI < 25 kg/m².

The rFSH and rhCG may be produced or expressed in a human cell line, preferably a PER.C6^{®} cell line. The rFSH and rhCG may be produced or expressed in a PER.C6^{®} cell line, a PER.C6^{®} derived cell line or a modified PER.C6^{®} cell line. rFSH which is produced or expressed in a PER.C6^{®} cell line will include some α2,6-linked sialic acids (α2,6 sialylation) provided by endogenous sialyl transferase activity [of the cell line] and will include some α2,3-linked sialic acids (α2,3 sialylation) provided by endogenous sialyl transferase activity. The cell line may be modified using α2,3-sialyltransferase. The cell line may be modified using α2,6-sialyltransferase. Alternatively or additionally, the rFSH may include α2,6-linked sialic acids (α2,6 sialylation) provided by endogenous sialyl transferase activity [of the cell line]. Herein, the term "human derived recombinant FSH" means recombinant FSH which is produced or expressed in a human cell line (e.g. recombinant FSH made by engineering a human cell line).

The composition may be a pharmaceutical composition. The pharmaceutical composition is for the treatment of infertility. The treatment of infertility may comprise COS prior to ART. The pharmaceutical composition may be used, for example, in medical indications where known FSH preparations are used.

The composition or pharmaceutical composition can be formulated into well-known compositions for any route of drug administration, e.g. oral, rectal, parenteral, transdermal (e.g. patch technology), intravenous, intramuscular, subcutaneous, intracisternal, intravaginal, intraperitoneal, local (powders, ointments or drops) or as a buccal or nasal spray. A typical composition comprises a pharmaceutically acceptable carrier, such as aqueous solution, nontoxic excipients, including salts and preservatives, buffers and the like, as described in Remington's Pharmaceutical Sciences fifteenth edition (Matt Publishing Company, 1975), at pages 1405 to 1412 and 1461 - 87, and the national formulary XIV fourteenth edition (American Pharmaceutical Association, 1975), among others.

Examples of suitable aqueous and non-aqueous pharmaceutical carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. The compositions of the present invention may also comprise additives such as but not limited to preservatives, wetting agents, emulsifying agents, surfactants and dispersing agents. Antibacterial and antifungal agents can be included to prevent growth of microbes and includes, for example, m-cresol, benzyl alcohol, paraben, chlorobutanol, phenol, sorbic acid, and the like. If a preservative is included, benzyl alcohol, phenol and/or m-cresol are preferred; however, the preservative is by no means limited to these examples. Furthermore, it may be desirable to include isotonic agents such as sugars, sodium chloride, amino acids and the like.

Preferably the composition or medicament comprises recombinant FSH and recombinant hCG and one or more of polysorbate 20, L-methionine, phenol, and arginine hydrochloride.

Injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use. Injectable formulations can be supplied in any suitable container, e.g. vial, pre-filled syringe, injection cartridges, and the like.

The composition or medicament may be formulated for single use or for multiple use (multiple dose). If the composition or medicament is formulated for multiple use, it is preferred that one or more preservatives is included. If a preservative is included, benzyl alcohol, phenol or m-cresol, are preferred; however, the preservative is by no means limited to these examples. The single use or multiple use formulated composition or medicament may further comprise an amino acid or combination of amino acids. Preferably the amino acid is arginine, for example added as arginine or more preferably arginine hydrochloride.

The composition or medicament may be included in a container such as a vial, prefilled cartridge (e.g. for single administration or multiple use) or an injection device such as a "pen" for e.g. administration of multiple doses.

The composition or pharmaceutical composition may be a formulation (e.g. injectable formulation) including FSH and hCG. The composition or medicament can be supplied in any appropriate package. For example, a composition or medicament can include a number of containers (e.g. pre-filled syringes or vials) containing both FSH and hCG. The syringes or vials may be packaged in a blister package or other means to maintain sterility. Any composition or medicament can optionally contain instructions for using the FSH and hCG formulation.

The pH and exact concentration of the various components of the pharmaceutical composition are adjusted in accordance with routine practice in this field. See GOODMAN and GILMAN's THE PHARMACOLOGICAL BASIS FOR THERAPEUTICES, 7th ed. In a preferred embodiment, the composition or medicament of the invention are supplied as compositions for parenteral administration. General methods for the preparation of the parenteral formulations are known in the art and are described in REMINGTON; THE SCIENCE AND PRACTICE OF PHARMACY, supra, at pages 780-820. The parenteral compositions can be supplied in liquid formulation or as a solid which will be mixed with a sterile injectable medium just prior to administration. In an especially preferred embodiment, the parenteral compositions are supplied in dosage unit form for ease of administration and uniformity of dosage.

According to the present invention in a further aspect there is provided a method of treatment of infertility in a (e.g. female) patient having serum AMH level <15 pmol/L comprising a step of administering to the patient a (e.g. single) composition comprising recombinant follicle stimulating hormone (FSH) and recombinant human chorionic gonadotropin (hCG), wherein the recombinant FSH is administered at a dose of, or equivalent to, 11 to 13 µg per day, and the recombinant hCG is administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example,12:1, 6:1, 3:1, 2:1, 1.5:1, 1:1 etc.. The recombinant FSH may be administered at a dose of 11 to 13 µg per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example, 12:1, 6:1, 3:1, 2:1, 1.5:1, 1:1 etc.. Preferably the ratio by weight of the dose per day of recombinant FSH to the dose per day of recombinant hCG (daily dose of rFSH in µg: daily dose of rhCG in µg) is 3:1 or 1.5:1. Preferably, the recombinant FSH and recombinant hCG are to be administered at doses per day of 12 µg recombinant FSH with 4 µg recombinant hCG; or 12 µg recombinant FSH with 8 µg recombinant hCG. The treatment of infertility may comprises a step of determining the serum AMH level of the patient, and a step of administering the doses of recombinant FSH and recombinant hCG to a patient having serum AMH level of <15 pmol/L.

According to the present invention in a further aspect there is provided a method of treatment of infertility in a (e.g. female) patient having serum AMH level of ≥15 pmol/L comprising a step of administering to the patient a (e.g. single) composition comprising recombinant follicle stimulating hormone (FSH) and recombinant human chorionic gonadotropin (hCG) wherein the composition is administered at a dose of, or equivalent to, 0.09 to 0.19 µg recombinant FSH per kg bodyweight of the patient per day, and the recombinant hCG is administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example,12:1, 6:1, 3:1, 2:1, 1.5:1, 1:1 etc.. The composition may be administered at a dose of 0.09 to 0.19 µg recombinant FSH per kg bodyweight of the patient per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example, 12:1, 6:1, 3:1, 2:1, 1.5:1, 1:1 etc.. The dose per day of recombinant FSH is preferably from a minimum dose of 6 µg to a maximum dose of 12 µg. Preferably, the ratio by weight of the dose per day of recombinant FSH to the dose per day of recombinant hCG is (daily dose of rFSH in µg: daily dose of rhCG in µg) is 3:1 or 1.5:1. The treatment of infertility may comprise a step of determining the serum AMH level of the patient, and a step of administering the doses of recombinant FSH and recombinant hCG to a patient having the defined serum AMH level.

According to the present invention in a further aspect there is provided the use of recombinant follicle stimulating hormone (FSH) and recombinant human chorionic gonadotropin (hCG) in the manufacture of a (single) medicament for the treatment of infertility in a patient having serum AMH level <15 pmol/L, wherein the recombinant FSH is to be administered at a dose of, or equivalent to, 11 to 13 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example,12:1, 6:1, 3:1, 2:1, 1.5:1, 1:1 etc.. The recombinant FSH may be administered at a dose of 11 to 13 µg per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example,12:1, 6:1, 3:1, 2:1, 1.5:1, 1:1 etc.. The ratio by weight of the dose per day of recombinant FSH to the dose per day of recombinant hCG (daily dose of rFSH in µg: daily dose of rhCG in µg) is preferably 3:1 or 1.5:1. Preferably the recombinant FSH and recombinant hCG are to be administered at doses per day of 12 µg recombinant FSH with 4 µg recombinant hCG; or 12 µg recombinant FSH with 8 µg recombinant hCG. The treatment of infertility may comprises a step of determining the serum AMH level of the patient, and a step of administering the doses of recombinant FSH and recombinant hCG to a patient having serum AMH level of <15 pmol/L.

According to the present invention in a further aspect there is provided the use of recombinant follicle stimulating hormone (FSH) and recombinant human chorionic gonadotropin (hCG) in the manufacture of a (single) medicament for the treatment of infertility in a patient having serum AMH level ≥15 pmol/L, wherein the composition is to be administered at a dose of, or equivalent to, 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example, 12:1, 6:1, 3:1, 2:1, 1.5:1, 1:1 etc.. The composition may be administered at a dose of 0.09 to 0.19 µg recombinant FSH per kg bodyweight of the patient per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1, for example 3:1 or 1.5:1. The ratio (daily dose of rFSH in µg: daily dose of rhCG in µg) may be, for example,12:1, 6:1, 3:1, 2:1, 1.5:1, 1:1 etc.. The dose per day of recombinant FSH may be from a minimum dose of 6 µg to a maximum dose of 12 µg. Preferably the ratio by weight of the dose per day of recombinant FSH to the dose per day of recombinant hCG is (daily dose of rFSH in µg: daily dose of rhCG in µg) is 3:1 or 1.5:1. The treatment of infertility may comprise a step of determining the serum AMH level of the patient, and a step of administering the doses of recombinant FSH and recombinant hCG to a patient having the defined serum AMH level.

The compositions of the present invention may be used, for example at higher doses, to treat patients who have undergone a previous course of treatment of infertility, or who for other reasons require higher doses of FSH.

Thus, according to the present invention in a further aspect there is provided a composition comprising recombinant follicle stimulating hormone (FSH) and recombinant human chorionic gonadotropin (hCG) for use in the treatment of infertility in a patient who has undergone a previous course of treatment of infertility (e.g. a previous course of treatment of infertility by controlled ovarian stimulation) in which 5 to 7 oocytes were retrieved (from the patient), wherein the recombinant FSH is to be administered at a dose of, or equivalent to, 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The recombinant FSH may be administered at a dose of 14 to 16 µg per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The ratio by weight of the dose per day of recombinant FSH to the dose per day of recombinant hCG (daily dose of rFSH in µg: daily dose of rhCG in µg) may be 3:1 or 1.5:1. The recombinant FSH may be administered at a dose of 15 µg per day, and the recombinant hCG administered at a dose of 5 µg per day or 7.5 µg per day.

According to the present invention in a further aspect there is provided a composition comprising recombinant follicle stimulating hormone (FSH) and recombinant human chorionic gonadotropin (hCG) for use in the treatment of infertility in a patient who has undergone a previous course of treatment of infertility (e.g. a previous course of treatment of infertility by controlled ovarian stimulation) in which 0 to 4 oocytes were retrieved (from the patient) or a previous course of treatment of infertility which was cancelled due to insufficient ovarian response, wherein the recombinant FSH is to be administered at a dose of, or equivalent to, 18 to 24 µg per day; and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The recombinant FSH may be administered at a dose of 18 to 24 µg per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The ratio by weight of the dose per day of recombinant FSH to the dose per day of recombinant hCG (daily dose of rFSH in µg: daily dose of rhCG in µg) may be 3:1 or 1.5:1. The recombinant FSH may be administered at a dose of 20 µg per day, and the recombinant hCG administered at a dose of 6.67 µg per day or 13.33 µg per day.

According to the present invention in a further aspect there is provided a composition comprising recombinant follicle stimulating hormone (FSH) and recombinant human chorionic gonadotropin (hCG) for use in the treatment of infertility in a patient, wherein the recombinant FSH is to be administered at a dose of, or equivalent to, 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.

According to the present invention in a further aspect there is provided a composition comprising recombinant follicle stimulating hormone (FSH) and recombinant human chorionic gonadotropin (hCG) for use in the treatment of infertility, wherein the recombinant FSH is to be administered at a dose of, or equivalent to, 18 to 24 µg per day; and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.

In these aspects of the invention the treatment of infertility may comprise a step of determining the serum AMH level of the patient, and a step of administering the doses of recombinant FSH and recombinant hCG to a patient having serum AMH level of <15 pmol/L. The recombinant FSH may include α2,6-sialylation and α2,3-sialylation, optionally wherein 1 % to 50% of the total sialylation is α2, 6-sialyation, and 50% to 99% of the total sialylation is α 2,3-sialyation. The recombinant hCG may include α2,6-sialylation and α2,3-sialylation, optionally wherein the hCG includes mono (1S), di (2S), tri (3S) and tetra (4S) sialylated structures. The patient may be over 30 years of age and/or have previously failed at least one cycle of infertility treatment. The treatment of infertility may promote good quality blastocysts (e.g. category 3BB or higher blastocysts, (i.e. 3BB, 3BA, 3AC, 3AB, 3AA, 4CC, 4CB,4CA, 4BC, 4BB, 4BA, 4AC, 4AB, 4AA, 5CC, 5CB, 5CA, 5BC, 5BB, 5BA, 5AC, 5AB, 5AA, 6CC,6CB, 6CA, 6BC, 6BB, 6BA, 6AC, 6AB, 6AA) e.g. increase the number of category 3BB or higher blastocysts on day 3 and/or day 5 after oocyte retrieval) or to improve embryo implantation.

These aspects of the invention include corresponding methods of treatment, and use of FSH and hCG in the manufacture of medicaments for use in corresponding treatments.

The compositions of the invention generally comprise single compositions comprising both FSH and hCG.

In other aspects of the invention, however, there are provided products comprising separate FSH and hCG compositions which may be for administration separately or together.

According to the present invention in a further aspect there is provided a product for use in the treatment of infertility in a patient having serum AMH level <15 pmol/L, the product including a composition comprising recombinant follicle stimulating hormone (FSH) and a (e.g. separate) composition including recombinant human chorionic gonadotropin (hCG), wherein the recombinant FSH is to be administered at a dose of, or equivalent to, 11 to 13 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The recombinant FSH may be administered at a dose of 11 to 13 µg per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The ratio by weight of the dose per day of recombinant FSH to the dose per day of recombinant hCG (daily dose of rFSH in µg: daily dose of rhCG in µg) may be 3:1 or 1.5:1. The recombinant FSH and recombinant hCG may be administered at doses per day of 12 µg recombinant FSH with 4 µg recombinant hCG; or 12 µg recombinant FSH with 8 µg recombinant hCG. The treatment of infertility may comprises a step of determining the serum AMH level of the patient, and a step of administering the doses of recombinant FSH and recombinant hCG to a patient having serum AMH level of <15 pmol/L.

According to the present invention in a further aspect there is provided a product for use in the treatment of infertility in a patient having serum AMH level ≥15 pmol/L, the product including a composition comprising recombinant follicle stimulating hormone (FSH) and a (e.g. separate) composition including recombinant human chorionic gonadotropin (hCG), wherein the recombinant FSH is to be administered at a dose of, or equivalent to, 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The rFSH may be administered at a dose of 0.09 to 0.19 µg recombinant FSH per kg bodyweight of the patient per day, and the recombinant hCG may be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The dose per day of recombinant FSH may be from a minimum dose of 6 µg to a maximum dose of 12 µg. The ratio by weight of the dose per day of recombinant FSH to the dose per day of recombinant hCG is (daily dose of rFSH in µg: daily dose of rhCG in µg) may be 3:1 or 1.5:1. The product may be for use in (i) the treatment of a patient having serum AMH level of 15 to 24.9 pmol/L, wherein the recombinant FSH is to be administered at a dose of 0.14 to 0.19 µg per kg bodyweight of the patient per day; or (ii) for use in the treatment of a patient having serum AMH level of 25 to 34.9 pmol/L, wherein the recombinant FSH is to be administered at a dose of 0.11 to 0.14 µg per kg bodyweight of the patient per day; or (iii) for use according in the treatment of a patient having serum AMH level of ≥ 35 pmol/L, wherein the recombinant FSH is to be administered at a dose of 0.10 to 0.11 µg recombinant FSH per kg bodyweight of the patient per day. The treatment of infertility may comprise a step of determining the serum AMH level of the patient, and a step of administering the doses of recombinant FSH and recombinant hCG to a patient having the defined serum AMH level.

According to the present invention in a further aspect there is provided a product for use in the treatment of infertility in a patient who has undergone a previous course of treatment of infertility (e.g. a previous course of treatment of infertility by controlled ovarian stimulation) in which 5 to 7 oocytes were retrieved (from the patient), the product including a composition comprising recombinant follicle stimulating hormone (FSH) and a (e.g. separate) composition including recombinant human chorionic gonadotropin (hCG), wherein the recombinant FSH is to be administered at a dose of, or equivalent to, 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The recombinant FSH may be administered at a dose of 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The ratio by weight of the dose per day of recombinant FSH to the dose per day of recombinant hCG (daily dose of rFSH in µg: daily dose of rhCG in µg) may be 3:1 or 1.5:1. The recombinant FSH may be administered at a dose of 15 µg per day, and the recombinant hCG may be administered at a dose of 5 µg per day or 7.5 µg per day. The treatment of infertility may comprise a step of determining the serum AMH level of the patient, and a step of administering the doses of recombinant FSH and recombinant hCG to a patient having serum AMH level of <15 pmol/L.

According to the present invention in a further aspect there is provided a product for use in the treatment of infertility in a patient who has undergone a previous course of treatment of infertility (e.g. a previous course of treatment of infertility by controlled ovarian stimulation) in which 0 to 4 oocytes were retrieved (from the patient) or a previous course of treatment of infertility which was cancelled due to insufficient ovarian response, the product including a composition comprising recombinant follicle stimulating hormone (FSH) and a (e.g. separate) composition including recombinant human chorionic gonadotropin (hCG), wherein the recombinant FSH is to be administered at a dose of, or equivalent to, 18 to 24 µg per day; and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The recombinant FSH may be administered at a dose of 18 to 24 µg per day, and the recombinant hCG may be be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1. The ratio by weight of the dose per day of recombinant FSH to the dose per day of recombinant hCG (daily dose of rFSH in µg: daily dose of rhCG in µg) may be 3:1 or 1.5:1. The recombinant FSH may be be administered at a dose of 20 µg per day, and the recombinant hCG may be administered at a dose of 6.67 µg per day or 13.33 µg per day. The treatment of infertility may comprise a step of determining the serum AMH level of the patient, and a step of administering the doses of recombinant FSH and recombinant hCG to a patient having serum AMH level of <15 pmol/L.

According to the present invention in a further aspect there is provided a product for use in the treatment of infertility in a patient, the product including a composition comprising recombinant follicle stimulating hormone (FSH) and a (e.g. separate) composition including recombinant human chorionic gonadotropin (hCG), wherein the recombinant FSH is to be administered at a dose of, or equivalent to, 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.

According to the present invention in a further aspect there is provided a product for use in the treatment of infertility in a patient, the product including a composition comprising recombinant follicle stimulating hormone (FSH) and a (e.g. separate) composition including recombinant human chorionic gonadotropin (hCG), wherein the recombinant FSH is to be administered at a dose of, or equivalent to, 18 to 24 µg per day; and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.

The recombinant FSH may include α2,6-sialylation and α2,3-sialylation, optionally wherein 1 % to 50% of the total sialylation is α2, 6-sialyation, and 50% to 99% of the total sialylation is α 2,3-sialyation. The recombinant hCG may include α2,6-sialylation and α2,3-sialylation, optionally wherein the hCG includes mono (1S), di (2S), tri (3S) and tetra (4S) sialylated structures. The patient may be over 30 years of age and/or have previously failed at least one cycle of infertility treatment. The treatment of infertility may promote good quality blastocysts (e.g. category 3BB or higher blastocysts, (i.e. 3BB, 3BA, 3AC, 3AB, 3AA, 4CC, 4CB,4CA, 4BC, 4BB, 4BA, 4AC, 4AB, 4AA, 5CC, 5CB, 5CA, 5BC, 5BB, 5BA, 5AC, 5AB, 5AA, 6CC,6CB, 6CA, 6BC, 6BB, 6BA, 6AC, 6AB, 6AA) e.g. increase the number of category 3BB or higher blastocysts on day 3 and/or day 5 after oocyte retrieval) or to improve embryo implantation.

These aspects of the invention include corresponding methods of treatment, and use of FSH and hCG in the manufacture of medicaments for use in corresponding treatments.

### Definitions

Technical and scientific terms used herein have the meanings commonly understood by one of ordinary skill in the art of assisted reproductive technology to which the present invention pertains, unless otherwise defined. Reference is made herein to various methodologies known to those of ordinary skill in the art. Any suitable materials and/or methods known to those of ordinary skill in the art can be utilized in carrying out the present invention. However, specific materials and methods are described. Materials, reagents and the like to which reference is made in the following description and examples are obtainable from commercial sources, unless otherwise noted.

It is to be understood, that any definitions and terms herein defined is meant to have the same meaning and purpose in any of the aspects and embodiments of the invention unless explicitly otherwise stated not to.

As used herein, the singular forms "a," "an," and "the" designate both the singular and the plural, unless expressly stated to designate the singular only.

As used herein, the term "about" means that the number or range is not limited to the exact number or range set forth, but encompass ranges around the recited number or range as will be understood by persons of ordinary skill in the art depending on the context in which the number or range is used. Unless otherwise apparent from the context or convention in the art, "about" mean up to plus or minus 10% of the particular term.

Herein the terms "patient" and "subject" are used interchangeably.

A ratio of by weight of the dose per day of recombinant FSH to the dose per day of recombinant hCG (daily dose of rFSH in µg: daily dose of rhCG in µg) of 1.5:1 is the same as a ratio of by weight of the dose per day of recombinant FSH to the dose per day of recombinant hCG (daily dose of rFSH in µg: daily dose of rhCG in µg) 3:2. Thus a composition for use according to the invention wherein the recombinant FSH and recombinant hCG are to be administered at doses per day of 12 µg recombinant FSH with 8 µg recombinant hCG may be referred to herein as having a ratio of by weight of the dose per day of recombinant FSH to the dose per day of recombinant hCG (daily dose of rFSH in µg: daily dose of rhCG in µg) of 1.5:1 or 3:2.

The daily doses of rFSH and rhCG are specified in microgrammes which can be abbreviated to µg or mcg.

Herein the term "treatment of infertility" includes treatment of infertility by controlled ovarian stimulation (COS) or methods which include a step or stage of controlled ovarian stimulation (COS), for example in vitro fertilisation (IVF), or intracytoplasmic sperm injection (ICSI). The term "treatment of infertility" includes treatment of infertility in a subject having tubal or unexplained infertility, including treatment of infertility in a subject having endometriosis, for example stage I or stage II endometriosis, and/or in a subject with a partner with male factor infertility. The composition may be for (use in) the treatment of infertility (and/or for controlled ovarian stimulation) in a subject having endometriosis, for example in a subject having stage I or stage II endometriosis, as defined by The American Society for Reproductive Medicine (ASRM) classification system for the various stages of endometriosis, (stage IV most severe; stage I least severe) [American Society for Reproductive Medicine. Revised American Society for Reproductive Medicine classification of endometriosis: 1996. Fertil Steril 1997; 67,817 821.].

Herein the term "GnRH agonist" means gonadotropin-releasing hormone agonist. GnRH agonists are a class of medications that act as agonists of the gonadotropin-releasing hormone receptor (GnRH receptor), the biological target of gonadotropin-releasing hormone.

Herein the term "GnRH antagonist" means gonadotropin-releasing hormone antagonist. GnRH antagonists are a class of medications that antagonize the gonadotropin-releasing hormone receptor (GnRH receptor) and thus the action of gonadotropin-releasing hormone (GnRH).

The term "follicle" herein means an ovarian follicle which is a fluid-filled sac that contains an immature egg, or oocyte.

A blastocyst forms in the early development of a human (or other mammal). In humans, blastocyst formation begins about 5 days after fertilization. The use of blastocysts in (IVF) generally involves retrieval (harvesting) from the woman a number of oocytes resulting from a controlled ovarian stimulation cycle; fertilization (insemination of) one or more oocytes and culturing the fertilized egg (oocyte) for five days to form a blastocyst (i.e. allowing the fertilized oocyte to develop to the blastocyst stage); and implanting the blastocyst into the uterus.

In accordance with all aspects described herein, it is preferred that the treatment of infertility described herein, is or includes, a step of COS. The cause of infertility could be the woman's partner suffering from male infertility, although it will be appreciated that according to the present invention it is the woman (female) who is treated by COS.

A treatment of infertility as described herein may be for, and may be effective for, development of multiple follicles and pregnancy after fresh and/or cryopreserved embryo transfer in ovulatory women undergoing assisted reproductive technology (ART).

A treatment of infertility as described herein may be for, and may be effective for, promoting good quality blastocysts (e.g., category 3BB or higher blastocysts, e.g., treatment of infertility to increase the number of category 3BB or higher blastocysts on day 5 after oocyte retrieval) and/or to improve embryo implantation. Herein a "good-quality blastocyst" is defined as category 3BB or higher (i.e. 3BB, 3BA, 3AC, 3AB, 3AA, 4CC, 4CB,4CA, 4BC, 4BB, 4BA, 4AC, 4AB, 4AA, 5CC, 5CB, 5CA, 5BC, 5BB, 5BA, 5AC, 5AB, 5AA, 6CC,6CB, 6CA, 6BC, 6BB, 6BA, 6AC, 6AB, 6AA). The treatment of infertility may be treatment of infertility to increase the number of category 3BB or higher blastocysts on day 5 after oocyte retrieval (e.g., as compared to treatment with Rekovelle^{®} or GONAL-F^{®}). The treatment of infertility may be treatment of infertility to increase the number of fertilised (2PN) oocytes (e.g., as compared to treatment with Rekovelle^{®} or GONAL-F^{®}).

As used herein, "day one of treatment", also referred to as "day one of stimulation", refers to the first day that the dose of (e.g., recombinant) FSH is administered to the patient. Day one of treatment (stimulation) may take place on day 1, 2 or 3, for example on day 2 or day 3, of the patient's menstrual cycle. In other words, day one of treatment (stimulation) may be one, two or three days, for example two or three days, after the patient commences menstrual bleeding, consistent with usage of this term in clinical practice with GnRH antagonist or GnRH agonist protocols. The term "during treatment" means on a day or on days that FSH is being administered to the patient.

### Examples

### Example 1: A randomised, double-blind, placebo-controlled, parallel-group, dose-range trial to investigate the efficacy and safety of FE 999302 as an add-on treatment to follitropin delta (REKOVELLE^{®}) in women undergoing controlled ovarian stimulation in a long GnRH agonist protocol

FE 999302 is a recombinant human choriogonadotropin (rhCG) being developed by Ferring Pharmaceuticals and intended for treatment of infertility given as daily administrations in combination with recombinant human follicle stimulating hormone (rFSH). FE 999302 is produced in a human derived cell line, PER.C6^{®}, differentiating it from OVITRELLE^{®}, a recombinant hCG produced from Chinese hamster ovary (CHO) cells. The doses of FE 999302 administered are specified by mass since that is intended for the final product. FE 999302 is produced by the method described in International Patent Application No. PCT/GB2010/001854, published as WO2011/042688A. A phase I trial showed that single subcutaneous administration of 4-256 µg FE 999302, and repeated administration of 8 µg per day and 16 µg FE 999302 per day, were safe and well tolerated and did not cause any safety concerns. After single administration of rhCG in down-regulated men, mean AUC was 1.5-fold greater for FE 999302 than for rhCG expressed in CHO cells. Mean Cmax was similar for the two preparations. In accordance with the differences in AUC, the apparent clearance (i.e. the ratio CL/F) was lower for FE 999302 (CL/F 0.5 vs. 0.8 L/h), respectively, explained by a longer terminal half-life (47 vs. 32 hours). The apparent distribution volumes (Vz/F) were comparable. The concentrations of testosterone (reflecting production of testosterone as a response to hCG exposure) reflected the pharmacokinetic profiles with a slight delay, resulting in 59% higher AUC with FE 999302. The pharmacokinetic parameters for FE 999302 were comparable in men and women at doses of 125 and 128 µg, respectively.

### Materials and Methods

This is a phase 2, multicentre, double-blind, randomised, parallel-group dose-range trial to investigate the efficacy and safety of FE 999302 as add-on treatment to follitropin delta (REKOVELLE^{®}) in first or second cycle patients aged 30 to 42 years undergoing controlled ovarian stimulation following a long GnRH agonist protocol. The trial has been designed to demonstrate the added effect of FE 999302 on blastocyst quality and other clinically relevant indicators relating to the overall successful outcome of treatment. Blastocyst quality is regarded as the best indicator for designing a phase 3 trial where the desired outcome is an increase in pregnancy and/or cumulative pregnancy rates.

The purpose of the Phase II dose-range trial is to examined the efficacy and safety of FE999302 when administrated as an add-on treatment to follitropin delta for COS prior ART. Based on previous controlled trials in which FSH was supplemented with hCG for COS, it is anticipated that FE999302 treatment may improve the total number of good-quality blastocysts as well as implementation following fresh embryo transfer. Increasing the number of good-quality blastocysts and improving implementation will increase the change of pregnancy in the treatment cycle following fresh embryo transfer. A higher number of good-quality blastocysts will also result in more cryopreserved blastocysts with the potential for frozen embryo transfer, thus increasing the cumulative pregnancy rate for any given treatment cycle.

Since the therapeutic window of FE 999302 is relatively uncertain, this phase 2 dose-range trial is designed to investigate the efficacy and safety of a broad dose range of FE 999302 when administered in combination with follitropin delta for COS prior to ART. In this trial, COS will not be compromised as all eligible subjects will receive an individualised dose of follitropin delta based on serum AMH levels at screening or up to 3 months prior to screening and body weight at randomisation.

It is planned to randomise 600 subjects of whom approximately 500 subjects will be randomised to one of 5 doses of FE 999302 (1, 2, 4, 8, or 12 µg) and approximately 100 subjects to placebo. It is estimated that up to 660 subjects should be screened to achieve 600 subjects eligible for randomisation.

### Inclusion Criteria

1. Informed consent documents signed prior to screening evaluations.
2. In good physical and mental health as judged by the investigator.
3. Anti-Müllerian hormone (AMH) levels at screening of 5.0-35.0 pmol/L (as measured by Elecsys^{®} AMH Plus Immunoassay [Roche Diagnostics] at central laboratory).
4. Pre-menopausal women between the ages of 30 and 42 years. The subjects must be at least 30 years (including the 30th birthday) and no more than 42 years (up to the day before the 43rd birthday) when they sign the informed consent.
5. Infertile women diagnosed with tubal infertility, unexplained infertility, endometriosis stage I/II or with partners diagnosed with male factor infertility, eligible for in vitro fertilisation (IVF) and/or intracytoplasmic sperm injection (ICSI) using fresh or frozen ejaculated sperm from male partner or sperm donor.
6. Infertility for at least 1 year before screening for subjects <35 years or for at least 6 months for subjects ≥35 years (not applicable in case of tubal or severe male factor infertility).
7. The trial cycle will be the subject's first or second controlled ovarian stimulation cycle and the subject must be suitable for individualised treatment with follitropin delta based on body weight and AMH, i.e. a previous cycle with follitropin delta is not allowed.
8. Regular menstrual cycles of 24-35 days (both inclusive), presumed to be ovulatory.
9. Hysterosalpingography, hysteroscopy, saline infusion sonography, or transvaginal ultrasound documenting a uterus consistent with expected normal function (e.g. no evidence of clinically interfering uterine fibroids defined as submucous or intramural fibroids larger than 3 cm in diameter, no polyps, and no congenital structural abnormalities which are associated with a reduced chance of pregnancy) within 6 months prior to screening.
10. Transvaginal ultrasound documenting presence and adequate visualisation of both ovaries, without evidence of significant abnormality (e.g. enlarged ovaries which would contraindicate the use of gonadotropins), and normal adnexa (e.g. no hydrosalpinx) within 1 year prior to screening. Both ovaries must be accessible for oocyte retrieval.
11. Early follicular phase (cycle day 2-5) serum levels of FSH between 1 and 15 IU/L (measured at central laboratory).
12. Negative serum hepatitis B surface antigen (HBsAg), hepatitis C virus (HCV), and human immunodeficiency virus (HIV) antibody tests within 1 year prior to randomisation.
13. Body mass index (BMI) between 17.5 and 32.0 kg/m2 (both inclusive) at screening.
14. Down-regulation confirmed within 28 days after the first GnRH agonist administration.
15. Willing to accept ICSI regardless the cause of infertility.
16. If ≤37 years willing to accept single blastocyst transfer. If ≥38 years willing to accept transfer of a single good-quality blastocyst or transfer of maximum 2 blastocysts if no good-quality blastocyst is available.
17. Willing to accept transfer of maximum 2 blastocysts per transfer in cryopreserved cycleswith blastocysts originating from the trial cycle and conducted within 1 year after randomisation.

### Exclusion Criteria

1. Known polycystic ovary syndrome (PCOS) associated with anovulation or known endometriosis stage III-IV (defined by the revised American Society for Reproductive Medicine [ASRM] classification, 1996).
2. Poor response in a previous controlled ovarian stimulation cycle for IVF/ICSI, defined as development of <4 follicles with a diameter ≥12 mm.
3. Excessive ovarian response in a previous controlled ovarian stimulation cycle for IVF/ICSI, defined as development of ≥25 follicles with a diameter ≥12 mm.
4. One or more follicles ≥10 mm (including cysts) observed on the transvaginal ultrasound after down-regulation prior to randomisation on stimulation day 1 (puncture of cysts is allowed prior to randomisation).
5. Known history of recurrent miscarriage (defined as three consecutive losses after ultrasound confirmation of pregnancy [excl. ectopic pregnancy] and before week 24 of pregnancy).
6. Known abnormal karyotype of subject or of her partner/sperm donor, as applicable, depending on source of sperm used for insemination in this trial. In case partner sperm will be used and the sperm production is severely impaired (concentration <1 million/mL) normal karyotype, including no Y-chromosome microdeletion, must be documented.
7. Any known clinically significant systemic disease (e.g. insulin-dependent diabetes).
8. Known inherited or acquired thrombophilia disease.
9. Active arterial or venous thromboembolism or severe thrombophlebitis, or a history of these events.
10. Known porphyria.
11. Any known endocrine or metabolic abnormalities (pituitary, adrenal, pancreas, liver or kidney) with the exception of controlled thyroid function disease.
12. Known presence of anti-hCG antibodies (based on the information available in the subject's medical records; i.e. not based on the anti-FE 999302 antibody analyses conducted in the trial).
13. Known tumours of the ovary, breast, uterus, adrenal gland, pituitary or hypothalamus which would contraindicate the use of gonadotropins.
14. Known impairment of renal or hepatic function.
15. Any abnormal finding of clinical chemistry and haematology at screening or vital signs at randomisation, which is judged clinically relevant by the investigator and/or requires intervention.
16. Currently breast-feeding.
17. Undiagnosed vaginal bleeding.
18. Known abnormal cervical cytology of clinical significance observed within 3 years prior to screening (unless the clinical significance has been resolved).
19. Findings at the gynaecological examination at screening which preclude gonadotropin stimulation or are associated with a reduced chance of pregnancy, e.g. congenital uterine abnormalities or retained intrauterine device.
20. Pregnancy (negative pregnancy tests must be documented at screening and prior to start of down-regulation) or contraindication to pregnancy.
21. Known current active pelvic inflammatory disease.
22. Use of fertility modifiers during the last menstrual cycle before screening, including dehydroepiandrosterone (DHEA) or cycle programming with oral contraceptives, progestogen or estrogen preparations.
23. Use of hormonal preparations (except for thyroid medication) during the last menstrual cycle before screening.
24. Known history of chemotherapy (except for gestational conditions) or radiotherapy.
25. Current or past (within 1 year prior to screening) abuse of alcohol or drugs, and/or current (last month) intake of more than 14 units of alcohol per week.
26. Current or past (within 90 days prior to screening) smoking habit of more than 10 cigarettes per day.
27. Hypersensitivity to any active ingredient or excipients in the medicinal products used in the trial.
28. Previous participation in the trial (i.e. re-screening is not allowed).
29. Use of any non-registered investigational drugs during the last 90 days prior to screening.

### Primary end point:

Number of good-quality blastocysts on day 5 after oocyte retrieval (grade 3BB or higher)

### Secondary endpoints

- Subjects with at least one good-quality blastocyst on day 5 after oocyte retrieval (grade 3BB or higher)
- Subjects with at least two good-quality blastocysts on day 5 after oocyte retrieval (grade 3BB or higher)
- Number and quality of embryos on day 3 after oocyte retrieval
- Number and quality of blastocysts on day 5 after oocyte retrieval
- Changes in serum hormone levels from stimulation day 1 to stimulation day 6, stimulation day 8, end-of-stimulation (progesterone, 17-OH-progesterone, androstenedione, testosterone, estradiol, inhibin A, inhibin B, FSH, and LH), and oocyte retrieval (progesterone, 17-OH-progesterone, androstenedione, testosterone, estradiol)
- Number and size of follicles on stimulation day 6 and at end-of-stimulation
- Positive βhCG (positive serum βhCG test 13-15 days after transfer)
- Clinical pregnancy (at least one gestational sac 5-6 weeks after transfer)
- Vital pregnancy (at least one intrauterine gestational sac with fetal heart beat 5-6 weeks after transfer)
- Ongoing pregnancy (at least one intrauterine viable fetus 10-11 weeks after transfer)
- Number of oocytes retrieved
- Number of metaphase II oocytes
- Number of fertilised (2PN) oocytes
- Total gonadotropin dose and number of stimulation days
- Incidence of cycle cancellation
- Serum concentrations of FE 999302 on stimulation days 1, 6, and 8, and at end-of-stimulation
- Incidence of ovarian hyperstimulation syndrome (early or late, any grade)
- Incidence and intensity of adverse events
- Changes in circulating levels of clinical chemistry and haematology parameters
- Incidence and intensity of injection site reactions after FE 999302 administration (redness, pain, itching, swelling, and bruising) assessed by the subject during the stimulation period
- Incidence of treatment-induced anti-FE 999302 antibodies, overall as well as with neutralising capacity
- Incidence Incidence of multi-fetal gestation, biochemical pregnancy, spontaneous abortion, ectopic pregnancy (with and without medical/surgical intervention), and vanishing twins

Eligible subjects will start down-regulation with GnRH agonist (triptorelin acetate, GONAPEPTYL^{®}) 0.1 mg/day subcutaneously in the mid-luteal phase (i.e. cycle day 21-24) of their menstrual cycle. Ovarian stimulation will start after 14 days (±1 day) if pituitary down-regulation is confirmed (serum estradiol <50 pg/ml or 180 pmol/l; local laboratory), and treatment with GnRH agonist is continued until the end of the stimulation period.

On stimulation day 1, down-regulated subjects will be randomised to receive a fixed daily dose of 1, 2, 4, 8, or 12 µg of FE 999302 or placebo. All randomised subjects will also receive an individualised fixed daily dose of follitropin delta (see Table C), determined on the basis of their AMH level at screening (as measured by ELECSYS^{®} AMH Plus Immunoassay [Roche Diagnostics]) and their body weight at stimulation day 1, throughout the stimulation period.

During stimulation, subjects will be monitored by transvaginal ultrasound on stimulation days 1 and 6 and hereafter at least every second day. When the leading follicle reaches ≥15 mm, transvaginal ultrasound must be performed daily. Triggering of final follicular maturation will be done as soon as ≥3 follicles with a diameter ≥17 mm are observed. If there are <25 follicles with a diameter ≥12 mm, a single dose of hCG (OVITRELLE^{®}) 250 µg will be administered, but if there are ≥25 follicles with a diameter ≥12 mm, the cycle is to be cancelled.

Oocyte retrieval will take place 36h (±2h) after triggering of final follicular maturation. All inseminated oocytes will be incubated in an EMBRYOSCOPE^{®} (Vitrolife) for time-lapse monitoring and embryo development will be recorded up to the day of transfer. Assessment of embryo quality on day 3 after oocyte retrieval will be done locally. Assessment of blastocyst quality on day 5 after oocyte retrieval will be done locally and centrally and consist of assessment of three parameters: blastocyst expansion and hatching status (grade 1-6), blastocyst inner cell mass grading (grade A-D) and trophectoderm grading (grade A-D) as is well known in the art. Blastocysts will be given a numerical score by using the system of Gardner & Schoolcraft, as is well known in the art, with the addition of D-categories for inner cell mass and trophectoderm. The primary endpoint is based on the central assessment (done by two independent assessors).

For all subjects, blastocyst transfer is performed on day 5 after oocyte retrieval. Single blastocyst transfer is mandatory for subjects ≤37 years. In subjects≥38 years, the transfer policy depends on the quality of the available blastocysts, i.e. single blastocyst transfer if they have at least one good-quality blastocyst, and transfer of a maximum 2 blastocysts if they have no good quality blastocysts (if two blastocysts are available). Remaining blastocysts may be cryopreserved and used by the subject after completion of the trial, in accordance with local guidelines and regulations.

### FE 999302/Placebo Dosing Regimen

Subjects will be randomised to receive a daily dose of 1, 2, 4, 8, or 12 µg of FE 999302 or placebo as a subcutaneous injection (see Example 1). The daily dose will be fixed throughout the stimulation period. Dosing will continue until the criterion for triggering of final follicular maturation has been met. Subjects can be treated with FE 999302 for a maximum of 20 days.

### Follitropin Delta Dosing Regimen

Randomised subjects will have their individual dose determined on the basis of their AMH level at screening and their body weight at stimulation day 1. For subjects with low AMH (<15 pmol/L) the daily follitropin delta dose is 12 µg, irrespective of body weight. For subjects with high AMH (≥15 pmol/L) the daily follitropin delta dose is on a continuous scale ranging from 0.19 to 0.10 µg/kg, i.e. dependent on actual AMH and body weight.

The daily follitropin delta dose will be fixed throughout the stimulation period. The maximum allowed daily follitropin delta dose is 12 µg. Dosing will continue until the criterion for triggering of final follicular maturation has been met. Subjects can be treated with follitropin delta for a maximum of 20 days. Coasting is not allowed.

The complete follitropin delta dosing regimen is tabulated in detail in Table B below.

**Table B Follitropin Delta Dosing Regimen**

| ***AMH concentration (pmol*/*L)*** | ***Daily dose fixed throughout stimulation*** | ***Maximum daily dose*** |
|---|---|---|
| <15 | 12 µg | 12 µg |
| 15-16 | 0.19 µg/kg | 12 µg |
| 17 | 0.18 µg/kg | 12 µg |
| 18 | 0.17 µg/kg | 12 µg |
| 19-20 | 0.16 µg/kg | 12 µg |
| 21-22 | 0.15 µg/kg | 12 µg |
| 23-24 | 0.14 µg/kg | 12 µg |
| 25-27 | 0.13 µg/kg | 12 µg |
| 28-32 | 0.12 µg/kg | 12 µg |
| 33-39 | 0.11 µg/kg | 12 µg |
| ≥40 | 0.10 µg/kg | 12 µg |
| AMH concentration will be rounded off to integers. Subjects can be treated for a maximum of 20 days. | | |

Follitropin delta is administered as daily subcutaneous injection(s) in the abdomen. To minimise local injection site reactions, it is advisable to change injection site regularly, but it should always be on the subject's right side of the navel to allow for distinction between injection site reactions related to FE 999302 or placebo and those related to follitropin delta.

It will be appreciated from the above that certain patients (e.g. those with low AMH, those with high AMH and high bodyweight) will be administered FSH and hCG at doses per day of 12 µg recombinant FSH with 4 µg recombinant hCG; or 12 µg recombinant FSH with 8 µg recombinant hCG. In other words, certain patients will be dosed wherein the ratio by weight of the dose per day of recombinant FSH to the dose per day of recombinant hCG (daily dose of rFSH in µg: daily dose of rhCG in µg) is 3:1 or 1.5:1. It is expected that this trial will demonstrate that these doses and ratios are highly effective (e.g. in terms of increase in number of category BB or greater oocytes; implantation etc.) and represent a route to a single composition which will provide individualised treatment with FSH and hCG for any patient, whatever their AMH level and bodyweight.

It is also expected, based on Phase I results, that the inclusion of hCG in the composition (and treatment protocol) will mean the claimed compositions may also be safe and effective (e.g. at higher doses) to treat patients who had insufficient ovarian response in a previous treatment (e.g. a previous COS cycle or cycles). It is expected that inclusion of hCG will mean that these patients may effectively be treated by dosage of 15 or 20 µg FSH (together with hCG), which is rather less than the current maximum dose (24 µg FSH) used to treat these patients.

### EXAMPLE 2 - REKOVELLE^{®} and hCG combined composition

REKOVELLE^{®} is a recombinant FSH expressed in a PER.C6^{®} cell line engineered by the methods disclosed in WO2013/020996 and WO2009/127826A.

The Marketing Authorisation holder for REKOVELLE^{®} is Ferring Pharmaceuticals A/S of Kay Fiskers Plads 11, 2300 Copenhagen S, Denmark, and it is available in the UK from Ferring Pharmaceuticals of Drayton Hall, Church Road, West Drayton, UB7 7PS, UK.

The active substance in REKOVELLE^{®} is follitropin delta (FE999049). REKOVELLE^{®} is highly sialylated and includes α2,3- and α2,6- sialylation, with about 85% to 90% of the total sialylation being α2,3-sialylation and about 10% to 15% of the total sialylation being α2,6-sialylation.

REKOVELLE^{®} is a clear and colourless solution for injection (injection). One millilitre of solution contains 33.3 micrograms of follitropin delta in each millilitre of solution. The other ingredients are phenol, polysorbate 20, L-methionine, sodium sulphate decahydrate, disodium phosphate dodecahydrate, concentrated phosphoric acid, sodium hydroxide and water for injections.

FE999302 is a recombinant hCG expressed in a PER.C6^{®} cell line engineered by the methods disclosed in WO2011/042688A and WO2016/170113A. FE999302 is highly sialylated and includes α2,3- and α2,6- sialylation, and a mix of mono-, di, tri and tetra sialylated structures.

### Example 2A

FE 999302 may be added to the REKOVELLE^{®} solution in an FSH:hCG ratio of, for example, 3:1 or 1.5:1, by methods well known in the art. One millilitre of the combined solution may contain 100 µg recombinant FSH and 33.3 µg FE 999302 or one millilitre of the combined solution may contain 100 µg recombinant FSH and 66.7 µg FE 999302.

### Example 2B

In another example, a combined product of FE 999302 and recombinant FSH (e.g. recombinant FSH expressed in a PER.C6^{®} cell line engineered by the methods disclosed in WO2013/020996 and WO2009/127826A) may further contain phenol, polysorbate 20, L-methionine, arginine hydrochloride, hydrochloric acid, sodium hydroxide and water for injection.

An example composition is as follows (sodium hydroxide and hydrochloric acid is added to pH 6.8, the amount of active ingredient per mL is set out above depending on 3:1 or 1.5:1 ratio):
5.0 mg/ml phenol
0.005 mg/ml polysorbate 20
0.15 mg/ml L-methionine
150 mM L-arginine hydrochloride
Q.s. hydrochloric acid
Q.s sodium hydroxide
pH 6.8 (range 6.0-8.0)
water for injection

One millilitre of the solution may contain 100 µg recombinant FSH and 33.3 µg FE 999302 (ration FSH:hCG of 3:1) or one millilitre of the combined solution may contain 100 µg recombinant FSH and 66.7 µg FE 999302 (ration 1.5:1).

The example composition is suitable for administration according to the invention.

### References

Baenziger JU and Green ED. (1988). Pituitary glycoprotein hormone oligosaccharides: structure, synthesis and function of the asparagine-linked oligosaccharides on lutropin, follitropin and thyrotropin. Biochim Biophys Acta. 947(2), 287-306.
Dalpathado DS, Irungu J, Go EP, Butnev VY, Norton K, Bousfield GR, and Desaire H. (2006). Comparative glycomics of the glycoprotein follicle stimulating hormone: glycopeptide analysis of isolates from two mammalian species. Biochemistry. 45(28), 8665-8673.
Dewailly D, Andersen CY, Balen A, Broekmans F, Dilaver N, Fanchin R, Griesinger G, Kelsey TW, La Marca A, Lambalk C et al. The physiology and clinical utility of anti-Mullerian hormone in women. Hum Reprod Update 2014;20:370-385.
Dias JA, Van Roey P. (2001). Structural biology of human follitropin and its receptor. Arch Med Res. 32(6), 510-519
Fox KM, Dias JA, and Van Roey P. (2001). Three-dimensional structure of human follicle-stimulating hormone. Mol Endocrinol. 15(3), 378-89
Kagawa Y, Takasaki S, Utsumi J, Hosoi K, Shimizu H, Kochibe N, and Kobata A. (1988). Comparative study of the asparagine-linked sugar chains of natural human interferon-beta 1 and recombinant human interferon-beta 1 produced by three different mammalian cells. J Biol Chem. 263(33), 17508-17515.
Olivennes F, Howles CM, Borini A, Germond M, Trew G, Wikland M, Zegers-Hochschild F, Saunders H (2009) Individualizing FSH dose for assisted reproduction using a novel algorithm: the CONSORT study. Reprod Biomed Online. 2009 Feb;18(2):195-204.
Pierce JG, and Parsons TF (1981) Glycoprotein hormones: structure and function Annu Rev Biochem. 50, 465-495.
Rathnam P, and Saxena BB. (1975). Primary amino acid sequence of follicle-stimulating hormone from human pituitary glands. I. alpha subunit. J Biol Chem.;250(17):6735-6746.
Ryan RJ, Keutmann HT, Charlesworth MC, McCormick DJ, Milius RP, Calvo FO and Vutyavanich T. (1987). Structure-function relationships of gonadotropins. Recent Prog Horm Res.;43,:383-429.
Saxena BB and Rathnam P. (1976) Amino acid sequence of the beta subunit of follicle-stimulating hormone from human pituitary glands. J Biol Chem. 251(4), 993-1005
Takeuchi M, Takasaki S, Miyazaki H, Kato T, Hoshi S, Kochibe N, and Kobata A (1988). Comparative study of the asparagine-linked sugar chains of human erythropoietins purified from urine and the culture medium of recombinant Chinese hamster ovary cells. J Biol Chem. 263(8), 3657-3663.
Ulloa-Aguirre A, Midgley AR Jr, Beitins IZ, and Padmanabhan V. (1995). Follicle-stimulating isohormones: characterization and physiological relevance. Endocr Rev.16(6), 765-787.
Wide L, Naessén T, Sundström-Poromaa I, Eriksson K. (2007) Sulfonation and sialylation of gonadotropins in women during the menstrual cycle, after menopause, and with polycystic ovarian syndrome and in men. J Clin Endocrinol Metab.;92(11), 4410-4417.
Yding Andersen C, Westergaard LG, and van Wely M. (2004). FSH isoform composition of commercial gonadotrophin preparations: a neglected aspect? Reprod Biomed Online. 9(2), 231-236.

There have been disclosed hereinbefore the compositions, compositions for use, uses and methods defined by the following numbered paragraphs:
1. A composition comprising recombinant follicle stimulating hormone (FSH) and recombinant human chorionic gonadotropin (hCG) for use in the treatment of infertility in a patient having serum AMH level <15 pmol/L, wherein the recombinant FSH is to be administered at a dose of, or equivalent to, 11 to 13 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
2. A composition for use according to paragraph 1 wherein the recombinant FSH is to be administered at a dose of 11 to 13 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
3. A composition for use according to paragraph 1 or paragraph 2 wherein the ratio by weight of the dose per day of recombinant FSH to the dose per day of recombinant hCG (daily dose of rFSH in µg: daily dose of rhCG in µg) is 3:1 or 1.5:1.
4. A composition for use according to any preceding paragraph wherein the recombinant FSH and recombinant hCG are to be administered at doses per day of 12 µg recombinant FSH with 4 µg recombinant hCG; or 12 µg recombinant FSH with 8 µg recombinant hCG.
5. A composition for use according to any preceding paragraph wherein the treatment of infertility comprises a step of determining the serum AMH level of the patient, and a step of administering the doses of recombinant FSH and recombinant hCG to a patient having serum AMH level of <15 pmol/L.
6. A composition comprising recombinant follicle stimulating hormone (FSH) and recombinant human chorionic gonadotropin (hCG) for use in the treatment of infertility in a patient having serum AMH level of ≥15 pmol/L, wherein the composition is to be administered at a dose of, or equivalent to, 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
7. A composition for use according to paragraph 6 wherein the composition is to be administered at a dose of 0.09 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
8. A composition for use according to paragraph 6 or paragraph 7 wherein the dose per day of recombinant FSH is from a minimum dose of 6 µg to a maximum dose of 12 µg.
9. A composition for use according to any of paragraphs 6 to 8 wherein the ratio by weight of the dose per day of recombinant FSH to the dose per day of recombinant hCG is (daily dose of rFSH in µg: daily dose of rhCG in µg) is 3:1 or 1.5:1.
10. A composition for use according to any of paragraphs 6 to 9 in (i) the treatment of a patient having serum AMH level of 15 to 24.9 pmol/L, wherein the recombinant FSH is to be administered at a dose of 0.14 to 0.19 µg per kg bodyweight of the patient per day; or (ii) for use in the treatment of a patient having serum AMH level of 25 to 34.9 pmol/L, wherein the recombinant FSH is to be administered at a dose of 0.11 to 0.14 µg per kg bodyweight of the patient per day; or (iii) for use according in the treatment of a patient having serum AMH level of ≥ 35 pmol/L, wherein the recombinant FSH is to be administered at a dose of 0.10 to 0.11 µg recombinant FSH per kg bodyweight of the patient per day.
11. A composition for use according to any of paragraphs 6 to 10 wherein the treatment of infertility comprises a step of determining the serum AMH level of the patient, and a step of administering the doses of recombinant FSH and recombinant hCG to a patient having the defined serum AMH level.
12. A composition comprising recombinant follicle stimulating hormone (FSH) and recombinant human chorionic gonadotropin (hCG) for use in the treatment of infertility in a patient who has undergone a previous course of treatment of infertility (e.g. a previous course of treatment of infertility by controlled ovarian stimulation) in which 5 to 7 oocytes were retrieved (from the patient), wherein the recombinant FSH is to be administered at a dose of, or equivalent to, 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
13. A composition for use according to paragraph 12 wherein the recombinant FSH is to be administered at a dose of 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
14. A composition for use according to paragraph 12 or paragraph 13 wherein the ratio by weight of the dose per day of recombinant FSH to the dose per day of recombinant hCG (daily dose of rFSH in µg: daily dose of rhCG in µg) is 3:1 or 1.5:1.
15. A composition for use according to any of paragraphs 12 to 14 wherein the recombinant FSH is to be administered at a dose of 15 µg per day, and the recombinant hCG is to be administered at a dose of 5 µg per day or 7.5 µg per day.
16. A composition comprising recombinant follicle stimulating hormone (FSH) and recombinant human chorionic gonadotropin (hCG) for use in the treatment of infertility in a patient who has undergone a previous course of treatment of infertility (e.g. a previous course of treatment of infertility by controlled ovarian stimulation) in which 0 to 4 oocytes were retrieved (from the patient) or a previous course of treatment of infertility which was cancelled due to insufficient ovarian response, wherein the recombinant FSH is to be administered at a dose of, or equivalent to, 18 to 24 µg per day; and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
17. A composition for use according to paragraph 16 wherein the recombinant FSH is to be administered at a dose of 18 to 24 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
18. A composition for use according to paragraph 16 or paragraph 17 wherein the ratio by weight of the dose per day of recombinant FSH to the dose per day of recombinant hCG (daily dose of rFSH in µg: daily dose of rhCG in µg) is 3:1 or 1.5:1.
19. A composition for use according to any of paragraphs 16 to 18 wherein the recombinant FSH is to be administered at a dose of 20 µg per day, and the recombinant hCG is to be administered at a dose of 6.67 µg per day or 13.33 µg per day.
20. A composition for use according to any of paragraphs 12 to 19 wherein the treatment of infertility comprises a step of determining the serum AMH level of the patient, and a
   step of administering the doses of recombinant FSH and recombinant hCG to a patient having serum AMH level of <15 pmol/L.
21. A composition comprising recombinant follicle stimulating hormone (FSH) and recombinant human chorionic gonadotropin (hCG) for use in the treatment of infertility in a patient, wherein the recombinant FSH is to be administered at a dose of, or equivalent to, 14 to 16 µg per day, and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
22. A composition comprising recombinant follicle stimulating hormone (FSH) and recombinant human chorionic gonadotropin (hCG) for use in the treatment of infertility, wherein the recombinant FSH is to be administered at a dose of, or equivalent to, 18 to 24 µg per day; and the recombinant hCG is to be administered such that the ratio by weight of the dose per day of recombinant FSH to the dose per day of recombinant hCG (daily dose of rFSH in µg: daily dose of rhCG in µg) is from 15:1 to 1:1, for example 3:1 to 1.5:1.
23. A composition for use according to any preceding paragraph wherein the recombinant FSH includes α2,6-sialylation and α2,3-sialylation, optionally wherein 1 % to 50% of the total sialylation is α2, 6-sialyation, and 50% to 99% of the total sialylation is α 2,3-sialyation.
24. A composition for use according to any preceding paragraph wherein the recombinant hCG includes α2,6-sialylation and α2,3-sialylation, optionally wherein the hCG includes mono (1S), di (2S), tri (3S) and tetra (4S) sialylated structures.
25. A composition for use according to any preceding paragraph wherein the patient is over 30 years of age and/or has previously failed at least one cycle of infertility treatment.
26. A composition for use according to any preceding paragraph wherein the treatment of infertility is to promote good quality blastocysts (e.g. category 3BB or higher blastocysts, (i.e. 3BB, 3BA, 3AC, 3AB, 3AA, 4CC, 4CB,4CA, 4BC, 4BB, 4BA, 4AC, 4AB, 4AA, 5CC, 5CB, 5CA, 5BC, 5BB, 5BA, 5AC, 5AB, 5AA, 6CC,6CB, 6CA, 6BC, 6BB, 6BA, 6AC, 6AB, 6AA) e.g. treatment of infertility to increase the number of category 3BB or higher blastocysts on day 3 and/or day 5 after oocyte retrieval) or to improve embryo implantation.
27. A (e.g. single) pharmaceutical composition comprising recombinant follicle stimulating hormone (FSH) and recombinant human chorionic gonadotropin (hCG) wherein the ratio by weight of the recombinant FSH to the recombinant hCG ( amount of rFSH in µg: amount of rhCG in µg) is 3:1 or 1.5:1.

## Claims

1. A composition comprising recombinant follicle stimulating hormone (FSH) and recombinant human chorionic gonadotropin (hCG) for use in the treatment of infertility, wherein the recombinant FSH and the recombinant hCG are to be administered such that the ratio by weight of the dose per day of recombinant FSH to the dose per day of recombinant hCG (daily dose of rFSH in µg: daily dose of rhCG in µg) is from 15:1 to 3:1.

2. A composition comprising recombinant follicle stimulating hormone (FSH) and recombinant human chorionic gonadotropin (hCG) for use in the treatment of infertility, wherein the recombinant FSH and the recombinant hCG are to be administered such that the ratio by weight of the dose per day of recombinant FSH to the dose per day of recombinant hCG (daily dose of rFSH in µg: daily dose of rhCG in µg) is from 12:1 to 6:1.

3. A composition for use according to claim 1 or claim 2 wherein the recombinant hCG includes α2,6-sialylation and α2,3-sialylation, optionally wherein the hCG includes mono (1S), di (2S), tri (3S) and tetra (4S) sialylated structures.

4. A composition comprising recombinant follicle stimulating hormone (FSH) and recombinant human chorionic gonadotropin (hCG) for use in the treatment of infertility in a patient having serum AMH level of ≥15 pmol/L, wherein the recombinant hCG includes α2,6-sialylation and α2,3-sialylation, wherein the hCG includes mono (1S), di (2S), tri (3S) and tetra (4S) sialylated structures, and wherein the composition is to be administered at a dose of, or equivalent to, 0.12 to 0.19 µg recombinant FSH per kg bodyweight of the patient per day, and the recombinant hCG is to be administered such that the ratio by weight of the dose per day of recombinant FSH to the dose per day of recombinant hCG (daily dose of rFSH in µg: daily dose of rhCG in µg) is from 12:1 to 6:1.

5. A composition for use according to claim 4 wherein the treatment of infertility comprises a step of determining the serum AMH level of the patient, and a step of administering the doses of recombinant FSH and recombinant hCG to a patient having serum AMH level of ≥15 pmol/L.

6. A composition for use according to any preceding claim wherein the dose per day of recombinant FSH is from a minimum dose of 6 µg to a maximum dose of 24 µg.

7. A composition for use according to any preceding claim wherein the recombinant FSH includes α2,6-sialylation and α2,3-sialylation, optionally wherein 1 % to 50% of the total sialylation is α2, 6-sialyation, and 50% to 99% of the total sialylation is α 2,3-sialyation.

8. A composition for use according to any preceding claim wherein the patient is over 30 years of age and/or has previously failed at least one cycle of infertility treatment.

9. A composition for use according to any preceding claim wherein the treatment of infertility is to promote good quality blastocysts (e.g. category 3BB or higher blastocysts, (i.e. 3BB, 3BA, 3AC, 3AB, 3AA, 4CC, 4CB,4CA, 4BC, 4BB, 4BA, 4AC, 4AB, 4AA, 5CC, 5CB, 5CA, 5BC, 5BB, 5BA, 5AC, 5AB, 5AA, 6CC,6CB, 6CA, 6BC, 6BB, 6BA, 6AC, 6AB, 6AA) e.g. treatment of infertility to increase the number of category 3BB or higher blastocysts on day 3 and/or day 5 after oocyte retrieval) or to improve embryo implantation.

10. A (e.g. single) pharmaceutical composition comprising recombinant follicle stimulating hormone (FSH) and recombinant human chorionic gonadotropin (hCG) wherein the recombinant hCG includes α2,6-sialylation and α2,3-sialylation, wherein the hCG includes mono (1S), di (2S), tri (3S) and tetra (4S) sialylated structures, and wherein the ratio by weight of the recombinant FSH to the recombinant hCG (amount of rFSH in µg: amount of rhCG in µg) is from 15:1 to 3:1, for example 12:1 to 6:1.
